# EUROPÄISCHE PATENTSCHRIFT

(11) **EP 1 288 227 B1**
(45) Veröffentlichungstag und Bekanntmachung des Hinweises auf die Patenterteilung: **29.03.2006**
(21) Anmeldenummer: 02015042.1
(22) Anmeldetag: 05.07.2002
(51) Int. Cl.: C07K 14/47

(54) **Coaktivator von nukleären Rezeptoren**
Coactivator of nuclear receptors
Coactivateur de récepteurs nucléaires

(30) Priorität: 23.07.2001 DE 10135787
(43) Veröffentlichungstag der Anmeldung: 05.03.2003
(73) Patentinhaber: Schering AG, 13353 Berlin (DE)
(72) Erfinder: Obendorf, Maik, 99423 Weimar (DE); Wolf, Siegmund, 07745 Jena (DE)

(56) Entgegenhaltungen:
- NAGASE T ET AL: "Prediction of the coding sequences of unidentified human genes. XVI. THE COMPLETE SEQUENCES OF 150 NEW CDNA CLONES FROM BRAIN WHICH CODE FOR LARGE PROTEINS IN VITRO" DNA RESEARCH, UNIVERSAL ACADEMY PRESS, JP, Bd. 7, 28. Februar 2000 (2000-02-28), Seiten 65-73, XP000949814 ISSN: 1340-2838 -& DATABASE EBI [Online] SWALL; 1. Oktober 2000 (2000-10-01) "Hypothetical protein KIAA1380" Database accession no. Q9P2G7 XP002213693
- DATABASE EBI [Online] EMBL; 29. September 2000 (2000-09-29) "Homo sapiens cDNA: FLJ21338 fis, clone COL02572" Database accession no. AK024991 XP002213692
- JACKSON TREVOR R ET AL: "Cytohesins and centaurins: Mediators of PI 3-kinase-regulated Arf signaling." TRENDS IN BIOCHEMICAL SCIENCES, Bd. 25, Nr. 10, Oktober 2000 (2000-10), Seiten 489-495, XP002213684 ISSN: 0968-0004
- BROWN MEGAN T ET AL: "ASAP1, a phospholipid-dependent Arf GTPase-activating protein that associates with and is phosphorylated by Src." MOLECULAR AND CELLULAR BIOLOGY, Bd. 18, Nr. 12, Dezember 1998 (1998-12), Seiten 7038-7051, XP002213685 ISSN: 0270-7306
- KAM JEANELLE L ET AL: "Phosphoinositide-dependent activation of the ADP-ribosylation factor GTPase-activating protein ASAP1. Evidence for the pleckstrin homology domain functioning as an allosteric site." JOURNAL OF BIOLOGICAL CHEMISTRY, Bd. 275, Nr. 13, 31. März 2000 (2000-03-31), Seiten 9653-9663, XP002213686 ISSN: 0021-9258
- JACKSON TREVOR R ET AL: "ACAPs are Arf6 GTPase-activating proteins that function in the cell periphery." JOURNAL OF CELL BIOLOGY, Bd. 151, Nr. 3, 30. Oktober 2000 (2000-10-30), Seiten 627-638, XP002213687 ISSN: 0021-9525
- ANDREEV J ET AL: "Identification of a new Pyk2 target protein with Arf-GAP activity." MOLECULAR AND CELLULAR BIOLOGY, Bd. 19, Nr. 3, März 1999 (1999-03), Seiten 2338-2350, XP002145146 ISSN: 0270-7306
- BOSHANS RITA L ET AL: "ADP-ribosylation factor 6 regulates actin cytoskeleton remodeling in coordination with Rac1 and RhoA." MOLECULAR AND CELLULAR BIOLOGY, Bd. 20, Nr. 10, Mai 2000 (2000-05), Seiten 3685-3694, XP002213688 ISSN: 0270-7306
- VAN AELST LINDA ET AL: "Rho GTPases and signaling networks." GENES & DEVELOPMENT, Bd. 11, Nr. 18, 1997, Seiten 2295-2322, XP002213689 ISSN: 0890-9369
- MIURA KOICHI ET AL: "ARAP1: A point of convergence for Arf and Rho signaling." MOLECULAR CELL, Bd. 9, Nr. 1, Januar 2002 (2002-01), Seiten 109-119, XP002213690 January, 2002 ISSN: 1097-2765
- KRUGMANN S ET AL: "Identification of ARAP3, a novel PI3K effector regulating both Arf and Rho GTPases, by selective capture on phosphoinositide affinity matrices." MOLECULAR CELL, Bd. 9, Nr. 1, Januar 2002 (2002-01), Seiten 95-108, XP002213691 January, 2002 ISSN: 1097-2765

## Beschreibung

Die Erfindung betrifft Comodulatoren von nukleären Rezeptoren, diese kodierende Nukleinsaüren, und ihre Verwendung zur Bereitstellung neuer Medikamente.

Die Superfamilie der nukleären Rezeptoren, zu der etwa 50 verschiedene Proteine gehören, besteht aus einer Gruppe verwandter Transkriptionsfaktoren, die die Transkription des jeweiligen Zielgens in Abhängigkeit bestimmter, spezifischer Liganden steuert. Diese Familie kann nach bestimmten Kriterien wie z.B. Dimerisationsstatus, Art des Liganden oder Struktur des DNA-Bindeelementes, in mehrere Subfamilien unterteilt werden (Beato et al., 2000, Human Reproduct. Update, 6, 225-236). Charakteristisches Merkmal der nukleären Rezeptoren ist die übereinstimmende Struktur von funktionellen Domänen (mit den Bezeichnungen A bis F), bestehend aus einer stark variablen, nur schwach konservierten N-terminalen Region mit autonomer konstitutiver Aktivierungsfunktion (AF-1), einer stark konservierten DNA-Bindungsdomäne (DBD), die für die Erkennung von speziellen DNA-Bindeelementen verantwortlich ist und aus zwei Zinkfinger-Motiven besteht, einer variablen Schamierdomäne (Hinge) und einer konservierten multifunktionalen C-terminalen Ligandenbindungsdomäne (LBD) bestehend aus Dimerisations- und Liganden-abhängiger Transaktivierungsfunktion (AF-2). Im Anschluss daran folgt die am weitesten C-terminal gelegene Region, deren Funktion nicht bekannt ist und die bei Rezeptoren wie z.B. PR (Progesteron-Rezeptor), PPAR (Peroxisom proliferator-aktivierter Rezeptor) und RXR (Retinoid-X-Rezeptor) fehlt (Mangelsdorf & Evans, 1995; Cell, 83, 841-850; Robyr et al., 2000, Mol. Endocrinol., 14, 329-347). Für einige nukleäre Rezeptoren (z.B. AR) wurde nachgewiesen, dass die N-terminale Region in der Lage ist, mit der C-terminalen Region in Wechselwirkung zu treten (Brinkmann et al., 1999, J. Steroid Biochem. and Mol. Biol., 69, 307-313). Steroidhormonrezeptoren wie z.B. Estrogen- (ER), Progesteron- (PR), Glukokortikoid- (GR), Mineralokortikoid- (MR) und Androgenrezeptoren (AR) binden steroidale Liganden, wie die Progestine, die Estrogene, die Glukokortikoide, die Mineralokortikoide und Androgene, die sich alle von Pregnenolon ableiten. Die Bindung des Liganden an den NR aktiviert den Rezeptor und steuert die Expression entsprechender Zielgene.

Darüber hinaus wurde eine weitere Proteinklasse, die sog. Comodulatoren, identifiziert, die entweder bei Aktivierung (Coaktivatoren) bzw. Repression (Corepressoren) der Gentranskription als Brückenmoleküle zwischen dem Transkriptionsinitiationskomplex und den nukleären Rezeptoren eine wichtige Rolle spielen (McKenna et al., 1999, Endocr. Rev., 20, 321-347). Ein Coaktivator verstärkt die Rezeptorfunktion und interagiert in Anwesenheit eines Agonisten - nicht dagegen in Anwesenheit eines Antagonisten - direkt mit der Aktivierungsdomäne von nukleären Rezeptoren. Er interagiert außerdem auch mit dem basalen Transkriptionsapparat und verstärkt dagegen nicht von allein die basale Transkriptionsaktivität. Die meisten Comodulatoren interagieren mit Hilfe eines oder mehrerer LXXLL-Motive (NR-Boxes) in der Proteinsequenz mit der AF-2-Domäne von nukleären Rezeptoren. Jedoch wurden auch einige Comodulatoren beschrieben, die mit anderen Aminosäuren mit nukleären Rezeptoren interagieren können (Ding et al., 1998, Mol. Endocrinol., 12, 302-313). Darüber hinaus wurden viele Comodulatoren identifiziert, die in ähnlicher Weise mit mehreren verschiedenen nukleären Rezeptoren interagieren.

Für einen Estrogen-Rezeptor-Coaktivator, genannt A1 B1, konnte gezeigt werden, dass er in Brustkrebs- und Ovarkrebs-Zellinien erhöht exprimiert ist und vermutlich eine wichtige Rolle bei dem Verlauf von Steroidhormon-abhängigen Tumoren spielt (Anzick et al. 1997, Science 277,965-968).

Neben der direkten Beeinflußung der Steroidhormonrezeptoren durch Hormone oder Antihormone wäre die Modulation der Wechselwirkung von Comodulatoren mit den Steroidhormonrezeptoren ein weiterer Angriffspunkt für eine Therapie von hormonabhängigen Krankheiten. Das Problem ist daher einen neuen Comodulator zu finden, mit dessen Hilfe neue Arzneimittel bereitgestellt werden können.

Das Problem wurde gelöst durch die Bereitstellung von Nukleinsäuren ausgewählt aus der Gruppe bestehend aus
a. Nukleinsäuren kodierend für Polypeptide, weiche die in Seq ID NO 14, 15 und 18 dargestellten Aminosäuresequenzen umfassen.
b. Nukleinsäuren mit den in Seq ID NO 1, 3 und 13 dargestellten Nukleotidsequenzen,

Teilbereiche dieser Sequenzen sind in der Datenbank GenBank unter den Accession No AB037801, AK027280 und AK024991 publiziert worden. Diesen Teilstücken wurde keine Funktion zugeordnet.

Nukleinsäuren können einzel- oder doppelsträngige DNA, z.B. cDNA, oder RNA, z.B. mRNA, cRNA, pre-mRNA darstellen.

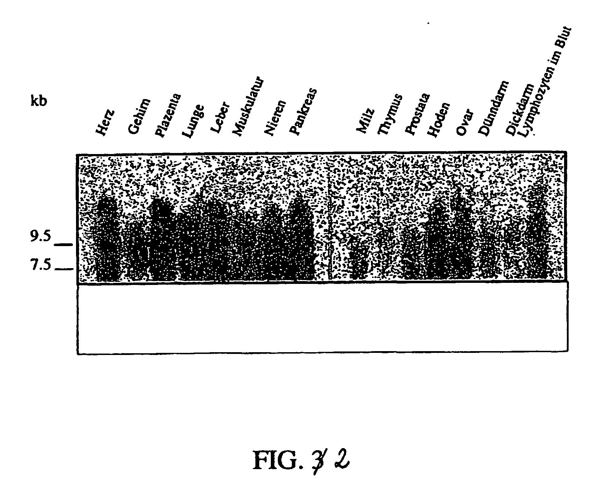

Die Erfindung betrifft weiterhin Polypeptide, die von den erfindungsgemäßen Nukleinsäuren kodiert werden und die in Seq ID NO 14, 15 und 18 dargestellte Aminosäuresequenzen umfassen. Diese erfindungsgemäßen Polypeptide werden im folgenden ARAP3 Varianten oder kurz ARAP3 genannt. Diese Nukleinsäurevarianten werden gewebsspezifisch durch Nutzung alternativer Promotoren exprimiert sowie durch alternatives Prozessieren bzw. Spleissen der pre mRNA erzeut. ARAP3 Varianten enthalten eine Zinkfinger-Domäne. Sie zeigen Homologien zu anderen Mitgliedern der Zinkfinger-Domänen-Familie wie z.B. Hairless -Protein (Cachon-Gonzales et al., 1994, Proc. Natl. Acad. Sci., 91, 7717-7721) und Testis Specific Protein a (Hoog et al., 1991, Mol. Reprod. Dev., 30, 173-181). Außerdem besteht für die C terminalen Aminosäuren eine signifikante Homologie zur jmjC Domäne der jumonji Familie (Balciunas and Ronne; 2000, Trends Biochem. Sci., 25, 274-76). Weiterhin enthält ARAP3 eine Kemlokalisationsdomäne. ARAP3 bindet an den Androgenrezeptor. Die Bindung erfolgt an den Bereich Aminosäure 325 bis 919 des Androgenrezeptors (s. FIG. 2 AR2-Fragment). Die Bindungstelle liegt in den Bereichen der erfindungsgemäßen Polypeptide, wie in der folgenden Tabelle angegeben:

**Tabelle 1 Aminosäurebereiche der Domänen von ARAP3 Polypeptiden**

| Seq ID NO | Zinkfinger AS | Androgenrezeptor Bindungsdomäne | Kemlokalisierungsdomäne (NLD) | jmjC - Domäne |
|---|---|---|---|---|
| 14 | 1664-1692 | 1732-1841 | 2183-2193 | 2199-2298 |
| 15 | 1858 bis 1886 | 1926 bis 2035 | 2377 bis 2385 | 2393 bis 2492 |
| 18 | 390 bis 418 | 458 bis 567 | 909 bis 917 | 925 bis 953 part. |

ARAP3 hat die Funktion eines Comodulators von nukleären Rezeptoren, besonders Steroidhormon-Rezeptoren. Beispiele für Rezeptoren sind der Androgen-Rezeptor, Estrogen-Rezeptor α, Estrogen-Rezeptor β, Progesteron Rezeptor A, Progesteron Rezeptor B, Glucocorticoid-Rezeptor, Mineratocorticoid-Rezeptor, Thyroid Hormon-Rezeptor, Vitamin D-Rezeptor und Peroxisom-Protiferator-activated Rezeptor. Besonders gut bindet ARAP3 an den Androgen-Rezeptor. Durch diese Bindung kann die Rezeptorfunktion modutiert werden, d.h. verstärkt oder geschwächt werden.

Im gesunden Menschen wird ARAP3 besonders stark im Herz, in der Leber, im Hoden und Ovar exprimiert.

Weiterhin sind Gegenstand der Erfindung Vektoren die mindestens eine Kopie einer der erfindungsgemäßen Nukleinsäuren enthalten. Vektoren können prokaryontische oder eukaryontische Vektoren sein. Beispiele für Vektoren sind pPRO (Clontech), pBAD (Invitrogen), pSG5 (Stratagene), pCl (Promega), plRES (Clontech), pBAC (Clontech), pMET (Invitrogen), pBlueBac (Invitrogen). In diese Vektoren können mit den dem Fachmann bekannten Methoden die erfindungsgemäßen Nukleinsäuren eingefügt werden. Die erfindungsgemäßen Nukleinsäuren befinden sich vorzugsweise in Verbindung mit Expressionssignalen wie z.B. Promotor und Enhancer auf dem Vektor.

Die Erfindung betrifft femer Zellen, die mit einer der erfindungsgemäßen Nukleinsäuresequenzen oder mit einem erfindungsgemäßen Vektor transfiziert sind. Als Zellen können z.B. *E. coli,* Hefe, *Pichia,* Sf9, COS, CV-1 oder BHK verwendet werden. Diese Zellen können für die Produktion der erfindungsgemäßen Polypeptide oder für Testsysteme verwendet werden.

Die erfindungsgemäßen Polypeptide oder Teilbereiche davon (Peptide) können zur Herstellung von Antikörpern verwendet werden. Zur Herstellung polyklonaler Antikörper können die Polypeptide oder Peptide, z.B. an KLH (Keyhole Limpet Hemocyanin), gebunden werden und Tieren, z.B. Kaninchen, gespritzt werden. Sie können auch zur Herstellung monoklonaler Antikörper verwendet werden. Zur Antikörperherstellung kann ein erfindungsgemäßes Polypeptid oder Peptid oder eine Mischung mehrerer erfindungsgemäßer Peptide verwendet werden. Die Herstellung der Antikörper erfolgt dabei nach Standardverfahren wie sie z.B. in Kohler, G. und Milstein, C., Nature 1975, 256, 495-497 und Nelson, P. N. et al., Mol. Pathol. 2000, 53, 111-117 beschrieben sind.

Gegenstand der Erfindung sind auch die Antikörper, die gegen die erfindungsgemäßen Polypeptide gerichtet sind.
Die erfindungsgemäßen Antikörper können zur Detektion von ARAP3 und seinen Varianten verwendet werden. Dies kann z.B. durch Immunhistochemie erfolgen. Die erfindungsgemäßen Antikörper können auch in anderen Immuntests, wie z.B. einem ELISA (enzyme linked immunosorbent assay) oder in Radioimmunotests, eingesetzt werden. So kann die Konzentration an ARAP3 in Gewebe-oder Zellextrakten nachgewiesen werden.

Der Nachweis der Expression der erfindungsgemäßen Polypeptide kann auch über den Nachweis von mRNA in den Zelten erfolgen. Gegenstand der Erfindung ist daher auch die Verwendung einer Sonde mit Nukleinsäuresequenzen, die komplementär zu den Nukleinsäuresequenzen sind, die für ARAP3 kodieren, zur Herstellung eines Reagenz zum Nachweis der Gegenwart von erfindungsgemäßer mRNA in Zellen. Eine Sonde ist ein kurzes DNA-Stück mit mindestens 14 Nukleotiden. Die erfindungsgemäßen Sonden können z.B. in einer Northem Blot Analyse verwendet werden. Diese Methode ist z.B. in Sambrook, J. et al., 1989, Cold Spring Harbor Laboratory Press, beschrieben. Andere Methoden zum Nachweis der RNA sind In Situ Hybridisierung, RNAse Protection Assay oder PCR.

Der Nachweis der Expression von ARAP3 wird vor allem bei Androgen-abhängigen Krankheiten eingesetzt. So kann z.B. eine erhöhte Androgen-Aktivierbarkeit bei Krankheiten wie Prostata-Krebs und benigner Prostata-Hyperplasie und bei Akne oder auch Haarausfall auf einer verstärkten Coaktivator-Aktivität von ARAP3 zurückzuführen sein. Eine verminderte Coaktivatoraktivität kann dagegen bei Hypogonadismus, erektiler Dysfunktion und Androgeninsensitiv-Syndromen, wie z.B. dem testikulären Feminisierungssyndrom, vorliegen. Bei diesen Krankheiten kann die Balance von Androgenrezeptor zu Coaktivator gestört sein. Daher ist es zweckmäßig neben der Menge an exprimiertem ARAP3 auch die Menge an exprimiertem Androgenrezeptor im gleichen Gewebe zu bestimmen. Das Androgenrezeptorprotein kann ebenfalls durch immunologische Methoden, wie z.B. Radioimmunoassay, ELlSA oder Westernblot, bestimmt werden.

Ein Gegenstand der Erfindung ist die Verwendung von ARAP3 oder der dafür kodierenden Nukleinsäuren als Zielsubstanz zur Herstellung eines Mittels zur Behandlung von Steroidhormon-abhängigen Erkrankungen. Solche Steroidhormon-abhängigen Erkrankungen können neben den oben erwähnten Androgen-abhängigen Erkrankungen auch z.B. Estrogen-abhängige Erkrankungen, wie z.B. Brustkrebs und Osteoporose, oder Herzkreislauferkrankungen und Gefäßerkrankungen sein. Auch könnte ARAP3 als Zielsubstanz für die Herstellung von Medikamenten zur Beeinflussung der männlichen Fertilität verwendet werden.

Insbesondere erfasst die Erfindung die Verwendung
a. einer erfindungsgemäßen Nukleinsäure,
b. eines erfindungsgemäßen Polypeptids, oder
c. einer erfindungsgemäßen Zelle
zur Identifizierung von Effektoren von ARAP3.
Effektoren sind Substanzen, die auf ARAP3 inhibitorisch oder aktivierend wirken, und die in der Lage sind, die Funktionen von ARAP3 zu beeinflussen, welche aufgrund der Wechselwirkungen durch Bindung von ARAP3 an ein Zielmolekül entstehen. Bevorzugt sind solche Substanzen, die die Interaktion von ARAP3 mit dem Androgenrezeptor modulieren. Dies kann z.B. getestet werden, indem die Bindung des gereinigten ARAP3-Polypeptids an das Androgenrezeptorpolypeptid in Gegenwart von zu testenden Substanzen gemessen wird und mit dem Kontrollwert, der ohne die zu testenden Substanzen erhoben wird, verglichen wird. Die Bindung kann durch Marker, die an ARAP3 oder am Androgenrezeptor gebunden sind, bestimmt werden. Solche Marker können z.B. Fluoreszenzmarker, Biotin oder radioaktive Marker sein.
Eine andere Möglichkeit zur Identifizierung von Effektoren ist die Verwendung einer Zelle, die cDNA von ARAP3 oder eines funktionellen Teils davon, die cDNA des Androgenrezeptors oder eines anderen nukleären Rezeptors und die cDNA eines Reportergens enthält. Als Reportergen kann z.B. Luziferase verwendet werden. Die Aktivität der Luziferase spiegelt hierbei die Aktivierung des nukleären Rezeptors wieder. Die Zellen werden in Gegenwart der zu testenden Substanzen inkubiert und die Luziferaseaktivität bestimmt. Die Inkubation kann auch zusätzlich in Gegenwart des Liganden des nukleären Rezeptors erfolgen. So können z.B. antagonistische Effekte gemessen werden. Bevorzugt ist die Verwendung des Androgenrezepors und dessen Liganden, einem Androgen.

Die Effektoren von ARAP3 können zur Behandlung von Steroidhormon-abhängigen Krankheiten eingesetzt werden. Bei Krankheiten, die z.B. auf zu einer starken Androgen-Aktivierbarkeit beruhen, kann ein Inhibitor der Interaktion von ARAP3 und dem Androgenrezeptor verabreicht werden und bei Krankheiten, die auf einer verminderten Coaktivator-Aktivität beruhen kann ein Stimulator dieser Interaktion verabreicht werden.

Krankheiten, die auf einem Mangel an ARAP3 beruhen, können auch durch eine Änderung der Konzentration von ARAP3 in den betroffenen Geweben behandelt werden. Dazu kann entweder eine erfindungsgemäße Nukleinsäure mit Hilfe eines in der Gentherapie gebräuchlichen Vektors oder ein erfindungsgemäßes Polypeptid in das Gewebe gebracht werden. Bei der Gentherapie wird ein Vektor, der eine erfindungsgemäße Nukleinsäure enthält, konstruiert und appliziert. Beispiele sind Vektoren, die aus Adenovirus. Adenovirusassociated virus, Herpes simplex virus oder SV40 abgeleitet sind. Die Gentherapie kann nach einem Protokoll wie von Gomez-Navarro, J. et al. (Eur. J. Cancer 1999, 35, 867-885) beschrieben durchgeführt werden. Die Applikation kann lokal, d.h. direkt, in das betroffene Gewebe, wie z.B. den Tumor, oder systemisch, d.h. über den Blutkreistauf erfolgen. Dies führt zu einer erhöhten Expression von ARAP3.

Die Applikation von ARAP3 kann in Form eines Fusionspolypeptids erfolgen. Durch das fusionierte Polypeptid, z.B. EGF oder Transferrin, wird das erfindungsgemäße Polypeptid bevorzugt zu dem gewünschten Gewebe, z.B. dem Tumorgewebe, transportiert.

Krankheiten können auch auf einer Überexpression von ARAP3 beruhen. Dadurch werden die nukleären Rezeptoren so stark sensitiviert, dass sie nicht nur durch ihre Liganden sondern auch durch andere Substanzen, die unter physiologischen Bedingungen keine Wirkung zeigen, aktiviert werden können. In solch einem Fall ist es wünschenswert, die Expression von ARAP3 zu emiedrigen. Dies kann durch Antisense-Moleküle erfolgen. Diese Moleküle sind komplementär zu den in Seq ID NO 1, 3 und 13 dargestellten Nukleinsäuresequenzen oder zu Teilen davon.
Weiterhin ist Gegenstand der Erfindung ein Verfahren zur Bereitstellung eines pharmazeutischen Mittels, wobei
a. Substanzen mit einem erfindungsgemäßen Testsystem in Kontakt gebracht werden,
b. die Wirkung der Substanzen auf das Testsystem im Vergleich zu Kontrollen gemessen wird,
c. eine Substanz, die in Schritt b. eine Modulation der Aktivität von ARAP3 zeigt, identifiziert wird
d. und die in Schritt c. identifizierte Substanz mit in der Pharmazie üblichen Formulierungsstoffen gemischt wird.

Die Aktivität von ARAP3, die in Schritt b. gemessen wird, ist die Verstärkung der Rezeptorfunktion des eingesetzten nukleären Rezeptors. Bevorzugt sind Steroidhormon-Rezeptoren, besonders bevorzugt ist der Androgenrezeptor.

Die erfindungsgemäßen Testsysteme können auch zur Testung von Umweltproben verwendet werden. Viele Substanzen in der Umwelt kommen in so niedrigen Konzentrationen vor, dass sie nur einen Effekt auf Steroidhormon-Rezeptoren der Menschen haben, die vermehrt den entsprechenden Coaktivator exprimieren. Durch Verwendung eines erfindungsgemäßen Testsystems könnten diese Substanzen identifiziert werden. Eine genetische Prädisposition für die Wirkung dieser Substanzen kann durch den erfindungsgemäßen Nachweis von ARAP3 bestimmt werden.

### Beschreibung der Abbildungen

FIG 1 zeigt eine schematische Darstellung des Androgenrezeptors. AF bedeutet Aktivierungsfunktion, DBD DNA-Bindungsdomäne, LBD Ligandenbindungsdomäne und AS steht für Aminosäure. Gezeigt ist das Fragment des Androgenrezeptors (AR2), welches für den Two-hybrid Screen verwendet wurde.
FIG 2 zeigt die Gewebsverteilung von ARAP3. Gezeigt ist eine Northernblot Analyse. 2µg humane Poly A+RNA wurden auf einem Gel aufgetrennt, auf eine Membran transferiert und mit einem ARAP3-cDNA-Fragment hybridisiert. kb steht für kilobasen.

### Beispiele

### Beispiel 1: Two-hybrid Screen

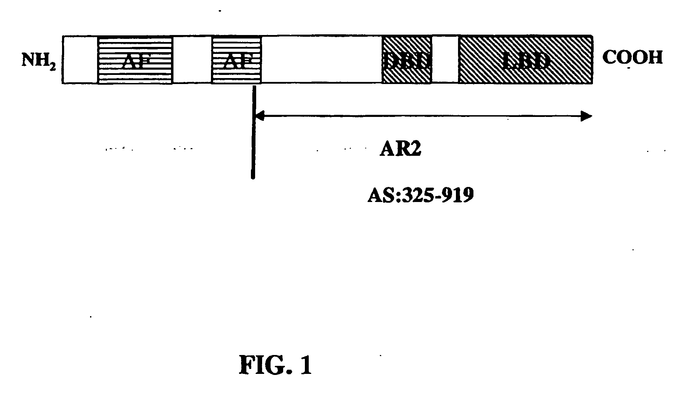

Unter Verwendung einer cDNA-Library aus fetalem Gehim (Clontech MATCHMAKER) und eines humanen AR-Fragments, das für die Aminosäuren 325 bis 919 kodiert, als Sonde (Abbildung 1) wurde ein Screening mittels Hefe-2-Hybrid System in Anwesenheit und Abwesenheit von 10⁻⁶ mol Dihydroxytestosteron (DHT) durchgeführt. In Übereinstimmung mit den Anweisungen des Herstellers (Clontech) betrug die Zahl der gescreenten Klone 3x10⁶ bzw. 2x10⁷. Die Zahl der unabhängigen Klone betrug laut Angaben des Herstellers 3.5x10⁶. Davon wurden 800 positive Klone ausgewählt und mit einem β-Galaktosidase-Assay getestet, wobei 240 als lacZ-positive Klone bestätigt wurden. Die Inserts dieser Klone wurden durch PCR amplifiziert. Mittels Restriktionsfragmentanalysen und Sequenzierung wurden mindestens 17 verschiedene Klone identifiziert. Einer davon war ein Klon mit einem 327 bp umfassenden Insert, das für einen Teil des ORF (Open reading frame) von KIAA1380 kodiert (Genbank-Zugangsnummer AB037801). Dieser Klon wurde beim Screenen der Bibliothek mit und ohne 10⁻⁶ mol DHT vierzig Mal identifiziert.

Mit Hilfe von PCR Techniken wurde dann die ARAP3-cDNA verlängert Dabei wurden verschiedene Spleißvarianten gefunden, welche die Nukleinsäuren 1, 3 und 13 darstellen. Aus diesen leiten sich 3 Polypeptide mit den Aminosäresequenzen 14, 15 und 18 ab.

### Beispiel 2: Bestimmung der Comodulator-Aktivität von ARAP3

Ein eukaryotischer Expressionsvektor, z.B. pCMX, der die ARAP3 cDNA, welche das vollständige ARAP3-Protein oder einen funktionellen Teil kodiert, enthält (pCMX-ARAP3), wird in geeignete Zelllinien, z.B. SH-SY5Y oder PC3 transfektiert, zusammen mit pSG5AR und pMMTV-luc. Als Kontrolle dient ein Ansatz mit dem leeren Expressionsvektor pCMX. Die androgene Aktivität kann anhand der Aktivität des Reporters Luziferase analog zum obigen Beispiel bestimmt werden. Der Effekt von ARAP3 auf das androgene Signal ist bestimmbar durch Vergleich der Aktivität des Kontrollansatzes.

### Beispiel 3: Bestimmung der Bindung von ARAP3 an den Androaenrezeptor mittels Pulldown Experiment

Als GST Pulldown Experiment bezeichnet man eine Versuchsanordung, die es erlaubt, ein in-vitro exprimiertes GST Fusionsprotein an ein ebenso in-vitro exprimiertes und radioaktiv markiertes Protein zu binden, nachfolgend von nichtinteragierenden Proteinen zu trennen und die gebundenen Proteine nachzuweisen und als Maß für die Bindung anzusehen. Die Experimente wurden entsprechend wie bei Sambrook und Russel, Molecular Cloning, Volume 3, Chapter 18, Protokoll 3 (Seite 18.55 ff): Cold Spring Harbour Laboratory Press; New York 2001 angegeben, durchgeführt:

Für die Expression wurde ein ARAP3 Fragment (AS 1735 bis 1840 der Seq ID NO 14) als GST Fusionsprotein in den Vektor pGEX-KG (Pharmacia) kloniert und nach den Vorschriften des Herstellers in Bakterienzellen überexprimiert und präpariert. Das ARAP3 Fusionsprotein wurde an Gtutathion Sepharose Kügelchen gebunden und mit 35S-Methionin markiertem Androgenrezeptor (pSG5AR mit T7 TNT Retikulozytenlysat der Fa. Promega exprimiert) inkubiert. Anschließend wurde zentrifugierend gewaschen und der gebundene Anteil von Androgenrezeptor an den GST-ARAP3 Kügelchen durch SDS-PAGE und anschließender Autofluografie bestimmt. Bei gleichartiger Behandlung von nichtfusionierten GST-Kügelchen gegenüber GST-ARAP3 Kügelchen in einem Versuchsansatz konnte durch Vergleich der Schwärzungen der Androgenrezeptor Proteinbanden auf dem Hyperfilm (TM Amersham) und Auswertung mittels Dichteintegration ein Bindungsverhältnis gebildet werden. Bezogen auf die durch Western Blot bestimmten Proteineinsatzmengen ergaben sich die validierten Bindungswerte von GST-ARAP3 gegenüber GST-leer, wie sie in Tabelle 2 als Ratio ARAP3 / GST dargestellt werden und als Nachweis für die Bindung von ARAP3 an den Androgenrezeptor anzusehen sind.

**Tabelle 2**

| Experiment | | Protein, Western | | | Bindung, Pulldown | | | RATIO |
|---|---|---|---|---|---|---|---|---|
| NO | | GST-leer In Vol./*µ*l | GST-ARAP3 In Vol./*µ*l | | GST-leer In Vol./*µ*l | GST-ARAP3 In Vol./*µ*l | | GST-ARAP3 /GST-leer |
| 1 | | 445 | 220 | | 160 | 2094 | | 26,5 |
| | | | | | | | | |
| 2 | | 833 | 351 | | 10,6 | -5000 | | 1122 |
| | | | | | | | | |
| 3 | | 4614 | 208 | | < 1 | 257 | | >> 100 |
| | | | | | | | | |
| 4 | | 349 | 382 | | < 1 | 225 | | >> 100 |

## Patentansprüche

1. Nukleinsäuren ausgewählt aus der Gruppe bestehend aus
a. Nukleinsäuren kodierend für Polypeptide mit den in Seq ID NO 14, 15 und 18 dargestellten Aminosäuresequenzen,
b. Nukleinsäuren mit den in Seq ID NO 1, 3 und 13 dargestellten Nukleotidsequenzen,
wobei die durch die Nukleinsäuren kodierten Polypeptide die Funktion eines Comodulators für nukleäre Rezeptoren besitzen.

2. Polypeptide kodiert von Nukleinsäuren nach Anspruch 1.

3. Polypeptide umfassend die in Seq ID NO 14, 15 und 18 dargestellten Aminosäuresequenzen.

4. Vektor, **dadurch gekennzeichnet, daß** er mindestens eine Kopie einer Nukleinsäure nach Anspruch 1 enthält.

5. Zelle, **dadurch gekennzeichnet, daß** sie mit einer Nukleinsäure nach Anspruch 1 oder mit einem Vektor nach Anspruch 4 transfiziert ist.

6. Verwendung einer Zelle nach Anspruch 5 zur Expression der Nukleinsäure nach Anspruch 1.

7. Verwendung eines Polypeptids nach Anspruch 2 oder 3 zur Herstellung von Antikörpern.

8. Verwendung einer Nukleinsäure nach Anspruch 1 oder eines Polypeptids nach Anspruch 2 oder 3 als Zielsubstanz zur Herstellung eines Mittels für Steroidhormon-abhängige Erkrankungen.

9. Verwendung
a. einer Nukleinsäure nach Anspruch 1,
b. eines Polypeptids nach Anspruch 2 oder 3,
c. einer Zelle nach Anspruch 5 zur Identifizierung von Effektoren eines Polypeptids nach Anspruch 2 oder 3.

10. Testsystem zur Detektion von Effektoren eines Polypeptids nach Anspruch 2 oder 3, wobei
a. in einer Zelle nach Anspruch 5 ein Reportergen exprimiert wird und
b. diese Zelle, wenn sie keinen oder nur wenig eines nukleären Rezeptors enthält, zusätzlich mit einem Vektor, der die DNA des nukleären Rezeptors enthält, transfiziert wird,
c. die Zelle in Gegenwart oder Abwesenheit der Testsubstanzen kultiviert wird und
d. die Veränderung der Expression des Reportergens gemessen wird.

11. Testsystem nach Anspruch 10, wobei der nukleäre Rezeptor der Androgenrezeptor ist.

12. Testsystem nach Anspruch 10, wobei die Zelle in Gegenwart oder Abwesenheit der Testsubstanzen bei gleichzeitiger Anwesenheit eines Liganden des nukleären Rezeptors kultiviert wird.

13. Testsystem nach Anspruch 12, wobei der nukleäre Rezeptor der Androgenrezeptor und der Ligand ein Androgen ist.

## Claims

1. Nucleic acids selected from the group consisting of
a) nucleic acids that code for polypeptides that have the amino acid sequences represented in Sequence ID Nos. 14, 15 and 18,
b) nucleic acids with the nucleotide sequences represented in Sequence ID Nos. 1, 3 and 13,
the polypeptides encoded by the nucleic acids possessing the function of a co-modulator for nuclear receptors.

2. Polypeptides encoded by nucleic acids according to Claim 1.

3. Polypeptides comprising the amino acid sequences represented in Sequence ID Nos. 14, 15 and 18.

4. Vector **characterized in that** it comprises at least one copy of a nucleic acid according to Claim 1.

5. Cell **characterized in that** it is transfected with a nucleic acid according to Claim 1 or with a vector according to Claim 4.

6. Use of a cell according to Claim 5 for expressing the nucleic acid according to Claim 1.

7. Use of a polypeptide according to Claim 2 or 3 for the production of antibodies.

8. Use of a nucleic acid according to Claim 1 or of a polypeptide according to Claim 2 or 3 as a target substance for preparing an agent for steroid hormone-dependent diseases.

9. Use
a) of a nucleic acid according to Claim 1,
b) of a polypeptide according to Claim 2 or 3,
c) of a cell according to Claim 5 for identifying effectors of a polypeptide according to Claim 2 or 3.

10. Assay system for detecting effectors of a polypeptide according to Claim 2 or 3, in which
a) a reporter gene is expressed in a cell according to Claim 5 and
b) this cell, if it contains no nuclear receptor or only little of a nuclear receptor, is additionally transfected with a vector containing the DNA of the nuclear receptor,
c) the cell is cultured in the presence or absence of the assay substances, and
d) the change in the expression of the reporter gene is measured.

11. Assay system according to Claim 10, the nuclear receptor being the androgen receptor.

12. Assay system according to Claim 10, the cell being cultured in the presence or absence of the assay substances in the simultaneous presence of a ligand of the nuclear receptor.

13. Assay system according to Claim 12, the nuclear receptor being the androgen receptor and the ligand being an androgen.

## Revendications

1. Acides nucléiques choisis dans le groupe consistant en
a. des acides nucléiques codant pour des polypeptides ayant les séquences d'aminoacides représentées dans SEQ ID n° 14, 15 et 18,
b. des acides nucléiques ayant les séquences nucléotidiques représentées dans SEQ ID n° 1, 3 et 13.
les polypeptides codés par les acides nucléiques ayant la fonction d'un co-modulateur pour des récepteurs nucléaires.

2. Polypeptides codés par les acides nucléiques selon la revendication 1.

3. Polypeptides comprenant les séquences d'aminoacides représentées dans SEQ ID n° 14, 15 et 18.

4. Vecteur, **caractérisé en ce qu'**il contient au moins une copie d'un acide nucléique selon la revendication 1.

5. Cellule, **caractérisée en ce qu'**elle est transfectée avec un acide nucléique selon la revendication 1 ou avec un vecteur selon la revendication 4.

6. Utilisation d'une cellule selon la revendication 5, pour l'expression de l'acide nucléique selon la revendication 1.

7. Utilisation d'un polypeptide selon la revendication 2 ou 3, pour la production d'anticorps.

8. Utilisation d'un acide nucléique selon la revendication 1 ou d'un polypeptide selon la revendication 2 ou 3, en tant que substance cible pour la préparation d'un agent pour des maladies dépendant d'hormones stéroïdiennes.

9. Utilisation
a. d'un acide nucléique selon la revendication 1,
b. d'un polypeptide selon la revendication 2 ou 3,
c. d'une cellule selon la revendication 5, pour l'identification d'effecteurs d'un polypeptide selon la revendication 2 ou 3.

10. Système d'essai pour la détection d'effecteurs d'un polypeptide selon la revendication 2 ou 3, dans lequel
a. un gène rapporteur est exprimé dans une cellule selon la revendication 5 et
b. cette cellule, si elle ne contient aucun récepteur nucléaire ou ne contient qu'un peu d'un récepteur nucléaire, est en outre transfectée avec un vecteur qui contient l'ADN du récepteur nucléaire,
c. la cellule est cultivée en présence ou en absence des substances d'essai et
d. on mesure la modification de l'expression du gène rapporteur.

11. Système d'essai selon la revendication 10, dans lequel le récepteur nucléaire est le récepteur d'androgène.

12. Système d'essai selon la revendication 10, dans lequel la cellule est cultivée en présence ou en absence des substances d'essai avec présence simultanée d'un ligand du récepteur nucléaire.

13. Système d'essai selon la revendication 12, dans lequel le récepteur nucléaire est le récepteur d'androgène et le ligand est un androgène.


### SEQUENZPROTOKOLL

<110> Jenapharm GmbH & Co. KG
<120> Comodulatoren von nukleären Rezeptoren
<130> 52145
<140>
   <141>
<160> 6
<170> PatentIn Ver. 2.1
<210> 1
   <211> 8734
   <212> DNA
   <213> Homo sapiens
<400> 1
   cacaaggctg cgaatctggg agtccaaagg agtgtttgcg gggagggccc gaggtcgctg 60
   tgtccggggc agagcggccg gttcgtcccg agtctgcgct ctttcgggtc cgctgctgtg 120
   tcccggtccg ctctcctcag catgagcggc cggtaggagt gaggtttcat cggttccctc 180
   gcactggagg aggcagcggc cgcttcggca gcgacagcta tggcggtaga gacgcgggca 240
   gagctggtgg gtaagcggtt cctgtgtgtg gcggtcggcg acgaggcacg ttcggagcgc 300
   tgggagagcg gacgcggctg gcgaagctgg cgagcggggg tcatccgagc cgtgtcacac 360
   agggacagcc gcaatccgga cctggcgatg attcaaataa gtggaaatgg aaactcttca 420
   ggtgtgtatg tggaatttga tgatcttgaa tgggataaac gagagtgggt taaagtttat 480
   gaagattttt caactttctt ggtggaatac cacttaatct gggccaaaag gaatgaccct 540
   agccagactc agggatcaaa gagcaaacag attcagtggc ctgcattgtt ctgggataca 600
   tatgcggaac gtgccggttt gttacataga ctttcaaacc tctggttgaa agaaatatac 660
   ccagttcagt cactgcagta gaattccttg tagataagca actggatttt ttaactgaag 720
   atagtgcctt tcagccctac caggacgaca tagacagcct aaacccagtt ctcagggaca 780
   acccgcagct tcatgaggaa gtgaaagtct gggtaaagga acaaaaggtt caggagattt 840
   ttatgcaagg tccttattcc ttaaatggat acagagtgag agtatataga caagactctg 900
   ccacccagtg gtttactggc ataattactc atcatgatct cttcacccgc accatgatcg 960
   ttatgaatga tcaggtacta gaaccacaga atgtcgatcc ttctatggtt caaatgacct 1020
   ttctagatga tgttgttcac tctttgttaa aaggtgaaaa tattggcatt acatcacgac 1080
   gcaggtctcg tgccaatcaa aacgtcaacg ctgttcacag ccattataca cgtgcccaag 1140
   caaatagtcc cagaccagca atgaactccc aagctgctgt accaaaacag aatacacacc 1200
   agcaacagca acaaagaagt atccgtccaa ataagaggaa gggctcagat agcagtatac 1260
   cagatgaaga gaagatgaag gaggaaaaat atgattatat atcacgagga gaaaatccta 1320
   aaggtaaaaa caaacacttg atgaataaaa gaaggaaacc tgaggaggat gaaaagaaac 1380
   taaatatgaa aagacttcga actgacaatg tttcagactt ttctgagagc agtgactcag 1440
   aaaattcaaa taagagaata atagataatt cctcagaaca gaagccagag aatgaattga 1500
   aaaataaaaa tacttcaaaa ataaatggag aagaaggaaa accccataat aatgagaagg 1560
   caggagaaga gaccctaaaa aatagccagc ctccctggga tcaaatacag gaagataaaa 1620
   aacatgaaga agcagagaag cggaagtctg ttgacactca gcttcaagaa gatatgatta 1680
   ttcattcgtc agaacagtcc acagtttctg atcataattc taatgattta cttcctcagg 1740
   aatgcaatat ggataaaaca cataccatgg aattgctacc aaaggagaag tttgtatcca 1800
   gaccacccac accaaaatgt gttattgata ttacaaatga cactaattta gaaaaggtgg 1860
   ctcaggaaaa ctcaagtacc tttggccttc agacacttca gaaaatggat cctaatgtta 1920
   gtgattcaaa acactctatt gcaaatgcaa aattcttgga aacagcaaaa aaagattctg 1980
   accagagctg ggtcagtgat gtagttaaag tggatctaac ccaatcaagt gttacaaatg 2040
   cttcttcagg aaatgatcac ttgaacatgg aaaaagagaa gtatgtctct tacatttctc 2100
   ctttaagtgc agtttctgtc atggaagata agctgcataa gcgaagtcca cctccagaga 2160
   ctataaaatc taaacttaat acttcagtag atactcacaa gataaaatcc agcccatcac 2220
   ctgaagttgt taaacccaaa ataactcatt ctcctgattc tgtaaagtct aaggccactt 2280
   atgtgaacag ccaagctact ggtgaaagaa gattggcaaa taagatagaa catgagctat 2340
   caagatgcag ttttcatcca attcctactc gaagcagtac attagaaact acaaagagtc 2400
   ctcttatcat tgataaaaat gagcatttta cagtttacag agatcctgca cttattgggt 2460
   cagaaacagg agctaatcat atttcacctt tcctaagcca gcatcctttt cctcttcact 2520
   cctcatctca tagaacctgt ttaaatccag gtacccatca tcctgcctta actcctgcac 2580
   cccatttact agccggatca tctagtcaaa ctccattacc taccattaac actcatcctc 2640
   tgactagtgg tccacaccat gctgttcatc accctcattt acttcccact gtgttacctg 2700
   gagtgcctac tgcctcctta cttggtggcc acccacgact agagagtgct catgccagca 2760
   gcttgagcca cttagcgcta gcacaccagc aacaacaaca gttgttacag caccagtcac 2820
   ctcatcttct tggacaagcc catccttctg cttcatataa tcagcttgga ctttatccaa 2880
   ttatttggca gtatccaaat ggaacacatg catactcagg acttggtttg ccttcttcta 2940
   agtgggttca cccagaaaat gcagttaatg ctgaagcttc attaaggagg aattctccca 3000
   gtccttggct acatcagccc acccctgtga cctcagcaga tggtattgga ttacttagtc 3060
   acattcctgt cagaccttcc agtgcagagc ctcatcggcc tcttaaaatt acagcccatt 3120
   ccagtccacc attgacaaaa actttagtag atcatcataa ggaagaatta gaaagaaaag 3180
   cttttatgga accattacgg tctgttgcat ccacatcagc caaaaatgac ctggatctaa 3240
   ataggtcaca gactggaaaa gattgtcact tacataggca ttttgtggat ccagtattaa 3300
   atcagttaca gaggccaccc caggagactg gagagaggtt aaacaaatac aaagaggaac 3360
   accgtcgaat tcttcaagaa agtattgatg ttgctccctt tacaactaaa atcaagggac 3420
   ttgagggtga gagagagaat tattccagag tggcatcatc atcttccagt cctaaaagcc 3480
   atatcatcaa acaagatatg gatgtagaac gctcagtatc agatctttat aaaatgaagc 3540
   actcagtgcc tcagagttta ccccaaagta actatttcac tacattgtct aatagtgtgg 3600
   tcaatgaacc accaagatca tacccatcca aagaagtttc aaatatttac ggtgataaac 3660
   agagtaatgc ccttgcagcg gcagcagcta atcctcaaac tctgacttca tttataacat 3720
   ctctttcaaa gcctccacct ttgattaaac accaaccaga aagtgaaggt ttagtaggca 3780
   agataccaga acatcttcca catcagattg catctcactc agtaacaacc ttcagaaatg 3840
   attgtaggag tcctacccat ttgacagttt cttctacaaa tacactccgc agtatgcctg 3900
   cattacatag agcaccagta tttcacccac caatccatca cagcctggaa agaaaggaag 3960
   gcagctatag tagtctttcc cctccaactt taactccggt gatgccagta aatgctggtg 4020
   gtaaagttca agaatcacag aagcctccaa ctctaatacc cgaaccaaaa gactcccagg 4080
   caaattttaa gagttcttca gaacagagtt tgacggagat gtggagacct aataataacc 4140
   tcagcaaaga gaaaactgaa tggcatgtgg agaaaagcag cggaaagtta caggctgcta 4200
   tggcatctgt cattgtgcgt ccatcttcta gtacaaaaac tgatagtatg ccagcaatgc 4260
   agttagcttc taaagatcga gttagtgaaa gatcttcagc tggggcacat aaaacagatt 4320
   gcctcaaact agcagaagcc ggagaaactg gaagaatcat tttgccaaat gtgaattcag 4380
   acagtgttca cacaaaatct gaaaaaaact ttcaggctgt ctcacagggc agtgttccca 4440
   gttcagtcat gtctgctgta aatacgatgt gtaataccaa aacggatgta atcacatctg 4500
   ctgccgatac taccagtgtt tccagctggg gtggttcaga agtaatttcc tctttatcaa 4560
   ataccatttt ggcctctaca tcatcagaat gtgtatcttc aaaaagtgtc agtcagccag 4620
   tggctcaaaa acaagaatgc aaggtcagca ccacagcacc agttacatta gccagtagta 4680
   agacaggaag tgttgttcaa cccagttctg ggttctcagg cacaactgat tttatccatt 4740
   taaaaaagca caaggcagca ttggctgcag ctcagtataa aagtagtaat gccagtgaga 4800
   ctgaacctaa tgctataaaa aatcagacac tttcagcctc ccttcctctg gatagcactg 4860
   taatctgtag tacaattaac aaagcaaact ctgtaggaaa tgggcaagct tcccagacaa 4920
   gtcaaccaaa ctaccatact aaactgaaaa aggcctggct caccagacac tcagaagaag 4980
   ataaaaatac taataaaatg gaaaattcag ggaattctgt atcagaaatt attaagccat 5040
   gttctgtcaa cttaatagcc tctacatcta gtgatataca aaatagtgta gatagtaaga 5100
   tcatagttga taaatatgta aaagatgata aagtcaacag gagaaaagcc aaaagaactt 5160
   atgaatctgg ctctgaaagt ggagactcag atgaaagtga aagcaagtca gagcaaagga 5220
   ctaaaagaca acctaagcca acttacaaaa agaagcaaaa tgatttgcaa aagagaaaag 5280
   gtgaaataga agaagatttg aaacccaatg gagttctcag caggagtgcc aaagaaagaa 5340
   gtaaactgaa gttgcaaagc aacagtaata ctggcattcc tcgttcagta ttgaaagatt 5400
   ggcgtaaagt caagaagctg aagcaaactg gggaatcctt tttacaggat gactcctgct 5460
   gtgagatagg gcctaattta caaaagtgtc gagaatgtag acttattcgc agtaaaaaag 5520
   gagaagaacc agctcactca ccagtatttt gtagatttta ctactttaga cggttgtcat 5580
   ttagtaaaaa cggagtagtt agaatagatg gtttctcttc tcctgaccaa tatgatgatg 5640
   aagctatgag tttgtggaca catgaaaatt ttgaagatga tgaactagat atagagactt 5700
   ctaaatatat cttggatata ataggtgata agttctgtca attagtaaca tctgaaaaaa 5760
   cagctttgtc ctgggtgaaa aaggatgcca aaattgcctg gaaaagagca gtgagaggag 5820
   tccgggagat gtgtgatgca tgtgaagcaa cattgtttaa cattcactgg gtctgccaaa 5880
   aatgtggatt tgtggtctgc ttagattgtt acaaggcaaa ggaaaggaag agttctagag 5940
   ataaagaact atatgcttgg atgaagtgtg tgaagggaca gcctcatgat cacaaacatt 6000
   taatgccaac ccaaattata cctggttctg ttttgacaga tcttctagat gccatgcaca 6060
   ctcttaggga aaaatatggt attaaatccc attgtcattg tactaacaaa cagaatttac 6120
   aagttggaaa ttttcctaca atgaatggtg tatctcaagt tttacagaat gttcttaatc 6180
   acagtaataa aatttctctg tgcatgcctg agtctcagca gcaaaatact cctccgaagt 6240
   ctgagaaaaa tggtggcagc agcccagaga gtgatgtagg cacagataac aagttaactc 6300
   ctccagaatc ccagtcacca ctgcactggt tagcagatct tgcagagcaa aaagccagag 6360
   aggaaaaaaa agaaaacaaa gaacttaccc ttgaaaacca aattaaagaa gaaagagaac 6420
   aagacaactc tgaatctcca aatggcagaa catcacctct tgtgtcccag aataatgaac 6480
   aaggctcaac cttacgggat ttgctgacta caacagctgg aaagctacgt gtggggtcta 6540
   cagatgctgg cattgccttt gccccagtat attcaatggg agccccaagt agcaaaagtg 6600
   gacggactat gcctaacatt cttgatgaca taattgcttc agttgttgaa aacaaaattc 6660
   caccaagtaa aacctccaag ataaatgtaa aaccagagct taaagaagag cctgaagaaa 6720
   gcataatatc tgcagtggat gaaaataata aattatacag tgatatacca cattcttgga 6780
   tctgtgagaa gcatatttta tggcttaagg attataagaa tagcagtaat tggaagcttt 6840
   tcaaagaatg ttggaaacaa ggacagcctg cagtggtttc tggtgtgcat aagaaaatga 6900
   acattagcct atggaaggcg gaatcaatta gtcttgattt tggagaccac caagctgatc 6960
   tcctgaactg caaagatagc atcatttcaa atgccaatgt taaggaattc tgggatggtt 7020
   ttgaagaagt ttcaaaacgg cagaaaaaca agagtggaga aacagttgtt ttaaaattga 7080
   aagactggcc ttcaggagaa gacttcaaga ctatgatgcc agcaagatac gaagatcttt 7140
   taaaaagtct gccattgcca gaatattgta atccagaagg aaaattcaat ttggcctctc 7200
   atttgccagg attttttgta cgtcctgatc taggacccag gttgtgcagt gcctatggtg 7260
   tagttgctgc taaagatcat gatataggaa caacaaatct ccatattgaa gtttctgatg 7320
   ttgtaaatat actagtttat gttggcatag caaaaggaaa tggcattctc tcaaaagcag 7380
   gaattctcaa gaaatttgag gaagaagatt tggatgacat tttaaggaaa agattgaagg 7440
   actcaagtga aatacctggt gctctgtggc atatttatgc tgggaaagat gttgacaaga 7500
   taagggaatt tcttcaaaag atttcaaaag aacaaggcct tgaagttcta ccagaacatg 7560
   atccaatacg tgaccaaagt tggtatgtga acaaaaagct ccgtcaaagg ctgcttgaag 7620
   aatatggagt cagaacctgt actcttattc agttccttgg tgatgctatt gttttgccag 7680
   cgggagcact tcatcaggtt cagaattttc acagctgtat tcaggtaact gaagattttg 7740
   tgtctccaga acatcttgta gagtcatttc atttaacaca ggaactgaga cttttgaagg 7800
   aagaaatcaa ttatgatgat aaactacagg ttaaaaatat tttgtatcat gcagtcaaag 7860
   aaatggtgag agccttgaag atacacgagg atgaagtaga ggatatggaa gaaaattaag 7920
   tgtgatccag tttgatattt ttaggttgtt gaactgggat tacttaacct tgaatgatga 7980
   tatgtatgca cactgacttt aagcttcata aaaccatcag tgccaagaaa ttctctttgt 8040
   agtaattact tgttactgac accacagcag tatagcatat gtcacagctc ctgtgattca 8100
   atgttataaa acaagcagaa ttttaaaagc agcactatat agctgttttg tattatagtg 8160
   tatatgatgt ttgtgaaaat gccagattta aaatgatgta tttatttttg gtaaaaaata 8220
   aaaaattcta tgctatattg ttgatcaagt gtaaatgtga ccttgtacag tttactaaaa 8280
   ttactgatat ttttcactac attgagacag ttactgtgag aataggacac aaacaccagc 8340
   tattgcctgc atctgggaaa ttgctgaatc gcacagcagt catgtcataa tcagaaaatt 8400
   actgccaaat aattgtaaaa tttgtaaagt ataaagtata taaagtagat actaaataca 8460
   gacacttcaa tattttgttg aagctattga ctgtacaatt aaacattttc aaaaggtgta 8520
   atttatttaa aattgtctca ttttggtaaa atttatgtga acttttaaag ctaaatatta 8580
   aacttaatat gctatgtaaa tatatacata tatacattta atgatgtatt tttttaaaac 8640
   attggcttgc ttttgttaaa gtgcaagtgt tacatatggc tttgtacatt aaagttgaaa 8700
   ggggttttac attttccatt aaaaggactt tatc 8734
<210> 3
   <211> 8656
   <212> DNA
   <213> Homo sapiens
<400> 3
<210> 13
   <211> 3781
   <212> DNA
   <213> Homo sapiens
<400> 13
   ctgatagtat gccagcaatg cagttagctt ctaaagatcg agttagtgaa agatcttcag 60
   ctggggcaca taaaacagat tgcctcaaac tagcagaagc cggagaaact ggaagaatca 120
   ttttgccaaa tgtgaattca gacagtgttc acacaaaatc tgaaaaaaac tttcaggctg 180
   tctcacaggg cagtgttccc agttcagtca tgtctgctgt aaatacgatg tgtaatacca 240
   aaacggatgt aatcacatct gctgccgata ctaccagtgt ttccagctgg ggtggttcag 300
   aagtaatttc ctctttatca aataccattt tggcctctac atcatcagaa tgtgtatctt 360
   caaaaagtgt cagtcagcca gtggctcaaa aacaagaatg caaggtcagc accacagcac 420
   cagttacatt agccagtagt aagacaggaa gtgttgttca acccagttct gggttctcag 480
   gcacaactga ttttatccat ttaaaaaagc acaaggcagc attggctgca gctcagtata 540
   aaagtagtaa tgccagtgag actgaaccta atgctataaa aaatcagaca ctttcagcct 600
   cccttcctct ggatagcact gtaatctgta gtacaattaa caaagcaaac tctgtaggaa 660
   atgggcaagc ttcccagaca agtcaaccaa actaccatac taaactgaaa aaggcctggc 720
   tcaccagaca ctcagaagaa gataaaaata ctaataaaat ggaaaattca gggaattctg 780
   tatcagaaat tattaagcca tgttctgtca acttaatagc ctctacatct agtgatatac 840
   aaaatagtgt agatagtaag atcatagttg ataaatatgt aaaagatgat aaagtcaaca 900
   ggagaaaagc caaaagaact tatgaatctg gctctgaaag tggagactca gatgaaagtg 960
   aaagcaagtc agagcaaagg actaaaagac aacctaagcc aacttacaaa aagaagcaaa 1020
   atgatttgca aaagagaaaa ggtgaaatag aagaagattt gaaacccaat ggagttctca 1080
   gcaggagtgc caaagaaaga agtaaactga agttgcaaag caacagtaat accaaaattg 1140
   cctggaaaag agcagtgaga ggagtccggg agatgtgtga tgcatgtgaa gcaacattgt 1200
   ttaacattca ctgggtctgc caaaaatgtg gatttgtggt ctgcttagat tgttacaagg 1260
   caaaggaaag gaagagttct agagataaag aactatatgc ttggatgaag tgtgtgaagg 1320
   gacagcctca tgatcacaaa catttaatgc caacccaaat tatacctggt tctgttttga 1380
   cagatcttct agatgccatg cacactctta gggaaaaata tggtattaaa tcccattgtc 1440
   attgtactaa caaacagaat ttacaagttg gaaattttcc tacaatgaat ggtgtatctc 1500
   aagttttaca gaatgttctt aatcacagta ataaaatttc tctgtgcatg cctgagtctc 1560
   agcagcaaaa tactcctccg aagtctgaga aaaatggtgg cagcagccca gagagtgatg 1620
   taggcacaga taacaagtta actcctccag aatcccagtc accactgcac tggttagcag 1680
   atcttgcaga gcaaaaagcc agagaggaaa aaaaagaaaa caaagaactt acccttgaaa 1740
   accaaattaa agaagaaaga gaacaagaca actctgaatc tccaaatggc agaacatcac 1800
   ctcttgtgtc ccagaataat gaacaaggct caaccttacg ggatttgctg actacaacag 1860
   ctggaaagct acgtgtgggg tctacagatg ctggcattgc ctttgcccca gtatattcaa 1920
   tgggagcccc aagtagcaaa agtggacgga ctatgcctaa cattcttgat gacataattg 1980
   cttcagttgt tgaaaacaaa attccaccaa gtaaaacctc caagataaat gtaaaaccag 2040
   agcttaaaga agagcctgaa gaaagcataa tatctgcagt ggatgaaaat aataaattat 2100
   acagtgatat accacattct tggatctgtg agaagcatat tttatggctt aaggattata 2160
   agaatagcag taattggaag cttttcaaag aatgttggaa acaaggacag cctgcagtgg 2220
   tttctggtgt gcataagaaa atgaacatta gcctatggaa ggcggaatca attagtcttg 2280
   attttggaga ccaccaagct gatctcctga actgcaaaga tagcatcatt tcaaatgcca 2340
   atgttaagga attctgggat ggttttgaag aagtttcaaa acggcagaaa aacaagagtg 2400
   gagaaacagt tgttttaaaa ttgaaagatt ggccttcagg agaagacttc aagactatga 2460
   tgccagcaag atacgaagat ctcttaaaaa gtctgccatt gccagaatat tgtaatccag 2520
   aaggaaaatt caatttggcc tctcatttgc caggattttt tgtacgtcct gatctaggac 2580
   ccaggttgtg cagtgcctat ggtgtagttg ctgctaaaga tcatgatata ggaacaacaa 2640
   atctccatat tgaagtttct gatgttgtaa atatactagt ttatgttggc atagcaaaag 2700
   gaaatggcat tctctcaaaa gcaggaattc tcaagaaatt tgaggaagaa gatttggatg 2760
   acattttaag gaaaagattg aaggactcaa gtgaaatacc tggtgctctg tggcatattt 2820
   atgctgggaa agatgttgac aagataaggg aatttcttca aaaggtataa gttagtttgc 2880
   aaataatatc tcttcccctc aagcaaggac ttattcattg ttgccgtacc ttcacttggc 2940
   attcaacaat ataagaccat tacataattt ttacttcaaa gtagatggga gtttttgttg 3000
   aaagtcaaaa ccctgtttct cttgctcctc tttgaaagtc tgttaacatt ttgagggttt 3060
   tgaatggtga tttgttccca gttggtagtg tgcctccatt ccctgttaat aaaagactga 3120
   tctgttcttg agccagatat tagttgggtg gcgaaaagta gctatctgga gtcgcgggat 3180
   gtaaaaacaa atatttcatg gaaacgtcta aagaaggtag gatctttatt ttctttgaat 3240
   ctgatagata tttttggctg ctgacctagg gaatgattct actctatcac atctgatact 3300
   atgatactta ctgagtagac tcagattttg agtaatcata ccttgactgt agttgtcata 3360
   tactctgaaa gaaatttata tatcaacctg aaataattgg ttgaaaccct ttgctcaggt 3420
   actttttaaa cttcgtaatt atggaaattt gttttaagaa atgttcagtg ctgatacatc 3480
   atctttctga aaggatgtaa gttctgtctg tgatcaatgt gaagtaaaag agttacagcc 3540
   ttttttgtac cattttatcc ctgaatactt acctgtattt taatctgaag tatgatcatt 3600
   tgtgccttct aaagcagatt atttaactga ttaaagagtc ccttgaaatt gatttttcaa 3660
   gcgttagaag gttagccatg taaaggtatt tatacttacc tcaaaaaagg atcaataaat 3720
   gtttatgaaa tccatacata tgaatggttt ggcttttggt aaaaaaaaaa aaaaaaaaaa 3780
   a                                                                 3781
<210> 14
   <211> 2358
   <212> PRT
   <213> Homo sapiens
<400> 14
   Met Gln Gly Pro Tyr Ser Leu Asn Gly Tyr Arg Val Arg Val Tyr Arg
   1               5                   10                  15
   Gln Asp Ser Ala Thr Gln Trp Phe Thr Gly Ile Ile Thr His His Asp
               20                  25                  30
   Leu Phe Thr Arg Thr Met Ile Val Met Asn Asp Gln Val Leu Glu Pro
           35                  40                  45
   Gln Asn Val Asp Pro Ser Met Val Gln Met Thr Phe Leu Asp Asp Val
       50                  55                  60
   Val His Ser Leu Leu Lys Gly Glu Asn Ile Gly Ile Thr Ser Arg Arg
   65                  70                  75                  80
   Arg Ser Arg Ala Asn Gln Asn Val Asn Ala Val His Ser His Tyr Thr
                   85                  90                  95
   Arg Ala Gln Ala Asn Ser Pro Arg Pro Ala Met Asn Ser Gln Ala Ala
               100                 105                 110
   Val Pro Lys Gln Asn Thr His Gln Gln Gln Gln Gln Arg Ser Ile Arg
           115                 120                 125
   Pro Asn Lys Arg Lys Gly Ser Asp Ser Ser Ile Pro Asp Glu Glu Lys
       130                 135                 140
   Met Lys Glu Glu Lys Tyr Asp Tyr Ile Ser Arg Gly Glu Asn Pro Lys
   145                 150                 155                 160
   Gly Lys Asn Lys His Leu Met Asn Lys Arg Arg Lys Pro Glu Glu Asp
                   165                 170                 175
   Glu Lys Lys Leu Asn Met Lys Arg Leu Arg Thr Asp Asn Val Ser Asp
               180                 185                 190
   Phe Ser Glu Ser Ser Asp Ser Glu Asn Ser Asn Lys Arg Ile Ile Asp
           195                 200                 205
   Asn Ser Ser Glu Gln Lys Pro Glu Asn Glu Leu Lys Asn Lys Asn Thr
       210                 215                 220
   Ser Lys Ile Asn Gly Glu Glu Gly Lys Pro His Asn Asn Glu Lys Ala
   225                 230                 235                 240
   Gly Glu Glu Thr Leu Lys Asn Ser Gln Pro Pro Trp Asp Gln Ile Gln
                   245                 250                 255
   Glu Asp Lys Lys His Glu Glu Ala Glu Lys Arg Lys Ser Val Asp Thr
               260                 265                 270
   Gln Leu Gln Glu Asp Met Ile Ile His Ser Ser Glu Gln Ser Thr Val
           275                 280                 285
   Ser Asp His Asn Ser Asn Asp Leu Leu Pro Gln Glu Cys Asn Met Asp
       290                 295                 300
   Lys Thr His Thr Met Glu Leu Leu Pro Lys Glu Lys Phe Val Ser Arg
   305                 310                 315                 320
   Pro Pro Thr Pro Lys Cys Val Ile Asp Ile Thr Asn Asp Thr Asn Leu
                   325                 330                 335
   Glu Lys Val Ala Gln Glu Asn Ser Ser Thr Phe Gly Leu Gln Thr Leu
               340                 345                 350
   Gln Lys Met Asp Pro Asn Val Ser Asp Ser Lys His Ser Ile Ala Asn
           355                 360                 365
   Ala Lys Phe Leu Glu Thr Ala Lys Lys Asp Ser Asp Gln Ser Trp Val
       370                 375                 380
   Ser Asp Val Val Lys Val Asp Leu Thr Gln Ser Ser Val Thr Asn Ala
   385                 390                 395                 400
   Ser Ser Gly Asn Asp His Leu Asn Met Glu Lys Glu Lys Tyr Val Ser
                   405                 410                 415
   Tyr Ile Ser Pro Leu Ser Ala Val Ser Val Met Glu Asp Lys Leu His
               420                 425                 430
   Lys Arg Ser Pro Pro Pro Glu Thr Ile Lys Ser Lys Leu Asn Thr Ser
           435                 440                 445
   Val Asp Thr His Lys Ile Lys Ser Ser Pro Ser Pro Glu Val Val Lys
       450                 455                 460
   Pro Lys Ile Thr His Ser Pro Asp Ser Val Lys Ser Lys Ala Thr Tyr
   465                 470                 475                 480
   Val Asn Ser Gln Ala Thr Gly Glu Arg Arg Leu Ala Asn Lys Ile Glu
                   485                 490                 495
   His Glu Leu Ser Arg Cys Ser Phe His Pro Ile Pro Thr Arg Ser Ser
               500                 505                 510
   Thr Leu Glu Thr Thr Lys Ser Pro Leu Ile Ile Asp Lys Asn Glu His
           515                 520                 525
   Phe Thr Val Tyr Arg Asp Pro Ala Leu Ile Gly Ser Glu Thr Gly Ala
       530                 535                 540
   Asn His Ile Ser Pro Phe Leu Ser Gln His Pro Phe Pro Leu His Ser
   545                 550                 555                 560
   Ser Ser His Arg Thr Cys Leu Asn Pro Gly Thr His His Pro Ala Leu
                   565                 570                 575
   Thr Pro Ala Pro His Leu Leu Ala Gly Ser Ser Ser Gln Thr Pro Leu
               580                 585                 590
   Pro Thr Ile Asn Thr His Pro Leu Thr Ser Gly Pro His His Ala Val
           595                 600                 605
   His His Pro His Leu Leu Pro Thr Val Leu Pro Gly Val Pro Thr Ala
       610                 615                 620
   Ser Leu Leu Gly Gly His Pro Arg Leu Glu Ser Ala His Ala Ser Ser
   625                 630                 635                 640
   Leu Ser His Leu Ala Leu Ala His Gln Gln Gln Gln Gln Leu Leu Gln
   645 650 655
   His Gln Ser Pro His Leu Leu Gly Gln Ala His Pro Ser Ala Ser Tyr
   660 665 670
   Asn Gln Leu Gly Leu Tyr Pro Ile Ile Trp Gln Tyr Pro Asn Gly Thr
   675 680 685
   His Ala Tyr Ser Gly Leu Gly Leu Pro Ser Ser Lys Trp Val His Pro
   690 695 700
   Glu Asn Ala Val Asn Ala Glu Ala Ser Leu Arg Arg Asn Ser Pro Ser
   705 710 715 720
   Pro Trp Leu His Gln Pro Thr Pro Val Thr Ser Ala Asp Gly Ile Gly
   725 730 735
   Leu Leu Ser His Ile Pro Val Arg Pro Ser Ser Ala Glu Pro His Arg
   740 745 750
   Pro Leu Lys Ile Thr Ala His Ser Ser Pro Pro Leu Thr Lys Thr Leu
   755 760 765
   Val Asp His His Lys Glu Glu Leu Glu Arg Lys Ala Phe Met Glu Pro
   770 775 780
   Leu Arg Ser Val Ala Ser Thr Ser Ala Lys Asn Asp Leu Asp Leu Asn
   785 790 795 800
   Arg Ser Gln Thr Gly Lys Asp Cys His Leu His Arg His Phe Val Asp
   805 810 815
   Pro Val Leu Asn Gln Leu Gln Arg Pro Pro Gln Glu Thr Gly Glu Arg
   820 825 830
   Leu Asn Lys Tyr Lys Glu Glu His Arg Arg Ile Leu Gln Glu Ser Ile
   835 840 845
   Asp Val Ala Pro Phe Thr Thr Lys Ile Lys Gly Leu Glu Gly Glu Arg
   850 855 860
   Glu Asn Tyr Ser Arg Val Ala Ser Ser Ser Ser Ser Pro Lys Ser His
   865 870 875 880
   Ile Ile Lys Gln Asp Met Asp Val Glu Arg Ser Val Ser Asp Leu Tyr
   885 890 895
   Lys Met Lys His Ser Val Pro Gln Ser Leu Pro Gln Ser Asn Tyr Phe
   900 905 910
   Thr Thr Leu Ser Asn Ser Val Val Asn Glu Pro Pro Arg Ser Tyr Pro
   915 920 925
   Ser Lys Glu Val Ser Asn Ile Tyr Gly Asp Lys Gln Ser Asn Ala Leu
   930 935 940
   Ala Ala Ala Ala Ala Asn Pro Gln Thr Leu Thr Ser Phe Ile Thr Ser
   945 950 955 960
   Leu Ser Lys Pro Pro Pro Leu Ile Lys His Gln Pro Glu Ser Glu Gly
   965 970 975
   Leu Val Gly Lys Ile Pro Glu His Leu Pro His Gln Ile Ala Ser His
                   980                 985                 990
   Ser Val Thr Thr Phe Arg Asn Asp Cys Arg Ser Pro Thr His Leu Thr
           995                 1000                1005
   Val Ser Ser Thr Asn Thr Leu Arg Ser Met Pro Ala Leu His Arg Ala
       1010                1015                1020
   Pro Val Phe His Pro Pro Ile His His Ser Leu Glu Arg Lys Glu Gly
   1025                1030                1035                1040
   Ser Tyr Ser Ser Leu Ser Pro Pro Thr Leu Thr Pro Val Met Pro Val
                   1045                1050                1055
   Asn Ala Gly Gly Lys Val Gln Glu Ser Gln Lys Pro Pro Thr Leu Ile
               1060                1065                1070
   Pro Glu Pro Lys Asp Ser Gln Ala Asn Phe Lys Ser Ser Ser Glu Gln
           1075                1080                1085
   Ser Leu Thr Glu Met Trp Arg Pro Asn Asn Asn Leu Ser Lys Glu Lys
       1090                1095                1100
   Thr Glu Trp His Val Glu Lys Ser Ser Gly Lys Leu Gln Ala Ala Met
   1105                1110                1115                1120
   Ala Ser Val Ile Val Arg Pro Ser Ser Ser Thr Lys Thr Asp Ser Met
                   1125                1130                1135
   Pro Ala Met Gln Leu Ala Ser Lys Asp Arg Val Ser Glu Arg Ser Ser
               1140                1145                1150
   Ala Gly Ala His Lys Thr Asp Cys Leu Lys Leu Ala Glu Ala Gly Glu
           1155                1160                1165
   Thr Gly Arg Ile Ile Leu Pro Asn Val Asn Ser Asp Ser Val His Thr
       1170                1175                1180
   Lys Ser Glu Lys Asn Phe Gln Ala Val Ser Gln Gly Ser Val Pro Ser
   1185                1190                1195                1200
   Ser Val Met Ser Ala Val Asn Thr Met Cys Asn Thr Lys Thr Asp Val
                   1205                1210                1215
   Ile Thr Ser Ala Ala Asp Thr Thr Ser Val Ser Ser Trp Gly Gly Ser
               1220                1225                1230
   Glu Val Ile Ser Ser Leu Ser Asn Thr Ile Leu Ala Ser Thr Ser Ser
           1235                1240                1245
   Glu Cys Val Ser Ser Lys Ser Val Ser Gln Pro Val Ala Gln Lys Gln
       1250                1255                1260
   Glu Cys Lys Val Ser Thr Thr Ala Pro Val Thr Leu Ala Ser Ser Lys
   1265                1270                1275                1280
   Thr Gly Ser Val Val Gln Pro Ser Ser Gly Phe Ser Gly Thr Thr Asp
                   1285                1290                1295
   Phe Ile His Leu Lys Lys His Lys Ala Ala Leu Ala Ala Ala Gln Tyr
               1300                1305                1310
   Lys Ser Ser Asn Ala Ser Glu Thr Glu Pro Asn Ala Ile Lys Asn Gln
   1315 1320 1325
   Thr Leu Ser Ala Ser Leu Pro Leu Asp Ser Thr Val Ile Cys Ser Thr
   1330 1335 1340
   Ile Asn Lys Ala Asn Ser Val Gly Asn Gly Gln Ala Ser Gln Thr Ser
   1345 1350 1355 1360
   Gln Pro Asn Tyr His Thr Lys Leu Lys Lys Ala Trp Leu Thr Arg His
   1365 1370 1375
   Ser Glu Glu Asp Lys Asn Thr Asn Lys Met Glu Asn Ser Gly Asn Ser
   1380 1385 1390
   Val Ser Glu Ile Ile Lys Pro Cys Ser Val Asn Leu Ile Ala Ser Thr
   1395 1400 1405
   Ser Ser Asp Ile Gln Asn Ser Val Asp Ser Lys Ile Ile Val Asp Lys
   1410 1415 1420
   Tyr Val Lys Asp Asp Lys Val Asn Arg Arg Lys Ala Lys Arg Thr Tyr
   1425 1430 1435 1440
   Glu Ser Gly Ser Glu Ser Gly Asp Ser Asp Glu Ser Glu Ser Lys Ser
   1445 1450 1455
   Glu Gln Arg Thr Lys Arg Gln Pro Lys Pro Thr Tyr Lys Lys Lys Gln
   1460 1465 1470
   Asn Asp Leu Gln Lys Arg Lys Gly Glu Ile Glu Glu Asp Leu Lys Pro
   1475 1480 1485
   Asn Gly Val Leu Ser Arg Ser Ala Lys Glu Arg Ser Lys Leu Lys Leu
   1490 1495 1500
   Gln Ser Asn Ser Asn Thr Gly Ile Pro Arg Ser Val Leu Lys Asp Trp
   1505 1510 1515 1520
   Arg Lys Val Lys Lys Leu Lys Gln Thr Gly Glu Ser Phe Leu Gln Asp
   1525 1530 1535
   Asp Ser Cys Cys Glu Ile Gly Pro Asn Leu Gln Lys Cys Arg Glu Cys
   1540 1545 1550
   Arg Leu Ile Arg Ser Lys Lys Gly Glu Glu Pro Ala His Ser Pro Val
   1555 1560 1565
   Phe Cys Arg Phe Tyr Tyr Phe Arg Arg Leu Ser Phe Ser Lys Asn Gly
   1570 1575 1580
   Val Val Arg Ile Asp Gly Phe Ser Ser Pro Asp Gln Tyr Asp Asp Glu
   1585 1590 1595 1600
   Ala Met Ser Leu Trp Thr His Glu Asn Phe Glu Asp Asp Glu Leu Asp
   1605 1610 1615
   Ile Glu Thr Ser Lys Tyr Ile Leu Asp Ile Ile Gly Asp Lys Phe Cys
   1620 1625 1630
   Gln Leu Val Thr Ser Glu Lys Thr Ala Leu Ser Trp Val Lys Lys Asp
   1635 1640 1645
   Ala Lys Ile Ala Trp Lys Arg Ala Val Arg Gly Val Arg Glu Met Cys
   1650 1655 1660
   Asp Ala Cys Glu Ala Thr Leu Phe Asn Ile His Trp Val Cys Gln Lys
   1665 1670 1675 1680
   Cys Gly Phe Val Val Cys Leu Asp Cys Tyr Lys Ala Lys Glu Arg Lys
   1685 1690 1695
   Ser Ser Arg Asp Lys Glu Leu Tyr Ala Trp Met Lys Cys Val Lys Gly
   1700 1705 1710
   Gln Pro His Asp His Lys His Leu Met Pro Thr Gln Ile Ile Pro Gly
   1715 1720 1725
   Ser Val Leu Thr Asp Leu Leu Asp Ala Met His Thr Leu Arg Glu Lys
   1730 1735 1740
   Tyr Gly Ile Lys Ser His Cys His Cys Thr Asn Lys Gln Asn Leu Gln
   1745 1750 1755 1760
   Val Gly Asn Phe Pro Thr Met Asn Gly Val Ser Gln Val Leu Gln Asn
   1765 1770 1775
   Val Leu Asn His Ser Asn Lys Ile Ser Leu Cys Met Pro Glu Ser Gln
   1780 1785 1790
   Gln Gln Asn Thr Pro Pro Lys Ser Glu Lys Asn Gly Gly Ser Ser Pro
   1795 1800 1805
   Glu Ser Asp Val Gly Thr Asp Asn Lys Leu Thr Pro Pro Glu Ser Gln
   1810 1815 1820
   Ser Pro Leu His Trp Leu Ala Asp Leu Ala Glu Gln Lys Ala Arg Glu
   1825 1830 1835 1840
   Glu Lys Lys Glu Asn Lys Glu Leu Thr Leu Glu Asn Gln Ile Lys Glu
   1845 1850 1855
   Glu Arg Glu Gln Asp Asn Ser Glu Ser Pro Asn Gly Arg Thr Ser Pro
   1860 1865 1870
   Leu Val Ser Gln Asn Asn Glu Gln Gly Ser Thr Leu Arg Asp Leu Leu
   1875 1880 1885
   Thr Thr Thr Ala Gly Lys Leu Arg Val Gly Ser Thr Asp Ala Gly Ile
   1890 1895 1900
   Ala Phe Ala Pro Val Tyr Ser Met Gly Ala Pro Ser Ser Lys Ser Gly
   1905 1910 1915 1920
   Arg Thr Met Pro Asn Ile Leu Asp Asp Ile Ile Ala Ser Val Val Glu
   1925 1930 1935
   Asn Lys Ile Pro Pro Ser Lys Thr Ser Lys Ile Asn Val Lys Pro Glu
   1940 1945 1950
   Leu Lys Glu Glu Pro Glu Glu Ser Ile Ile Ser Ala Val Asp Glu Asn
   1955 1960 1965
   Asn Lys Leu Tyr Ser Asp Ile Pro His Ser Trp Ile Cys Glu Lys His
   1970 1975 1980
   Ile Leu Trp Leu Lys Asp Tyr Lys Asn Ser Ser Asn Trp Lys Leu Phe
   1985 1990 1995 2000
   Lys Glu Cys Trp Lys Gln Gly Gln Pro Ala Val Val Ser Gly Val His
   2005 2010 2015
   Lys Lys Met Asn Ile Ser Leu Trp Lys Ala Glu Ser Ile Ser Leu Asp
   2020 2025 2030
   Phe Gly Asp His Gln Ala Asp Leu Leu Asn Cys Lys Asp Ser Ile Ile
   2035 2040 2045
   Ser Asn Ala Asn Val Lys Glu Phe Trp Asp Gly Phe Glu Glu Val Ser
   2050 2055 2060
   Lys Arg Gln Lys Asn Lys Ser Gly Glu Thr Val Val Leu Lys Leu Lys
   2065 2070 2075 2080
   Asp Trp Pro Ser Gly Glu Asp Phe Lys Thr Met Met Pro Ala Arg Tyr
   2085 2090 2095
   Glu Asp Leu Leu Lys Ser Leu Pro Leu Pro Glu Tyr Cys Asn Pro Glu
   2100 2105 2110
   Gly Lys Phe Asn Leu Ala Ser His Leu Pro Gly Phe Phe Val Arg Pro
   2115 2120 2125
   Asp Leu Gly Pro Arg Leu Cys Ser Ala Tyr Gly Val Val Ala Ala Lys
   2130 2135 2140
   Asp His Asp Ile Gly Thr Thr Asn Leu His Ile Glu Val Ser Asp Val
   2145 2150 2155 2160
   Val Asn Ile Leu Val Tyr Val Gly Ile Ala Lys Gly Asn Gly Ile Leu
   2165 2170 2175
   Ser Lys Ala Gly Ile Leu Lys Lys Phe Glu Glu Glu Asp Leu Asp Asp
   2180 2185 2190
   Ile Leu Arg Lys Arg Leu Lys Asp Ser Ser Glu Ile Pro Gly Ala Leu
   2195 2200 2205
   Trp His Ile Tyr Ala Gly Lys Asp Val Asp Lys Ile Arg Glu Phe Leu
   2210 2215 2220
   Gln Lys Ile Ser Lys Glu Gln Gly Leu Glu Val Leu Pro Glu His Asp
   2225 2230 2235 2240
   Pro Ile Arg Asp Gln Ser Trp Tyr Val Asn Lys Lys Leu Arg Gln Arg
   2245 2250 2255
   Leu Leu Glu Glu Tyr Gly Val Arg Thr Cys Thr Leu Ile Gln Phe Leu
   2260 2265 2270
   Gly Asp Ala Ile Val Leu Pro Ala Gly Ala Leu His Gln Val Gln Asn
   2275 2280 2285
   Phe His Ser Cys Ile Gln Val Thr Glu Asp Phe Val Ser Pro Glu His
   2290 2295 2300
   Leu Val Glu Ser Phe His Leu Thr Gln Glu Leu Arg Leu Leu Lys Glu
   2305 2310 2315 2320
   Glu Ile Asn Tyr Asp Asp Lys Leu Gln Val Lys Asn Ile Leu Tyr His
   2325 2330 2335
   Ala Val Lys Glu Met Val Arg Ala Leu Lys Ile His Glu Asp Glu Val
   2340 2345 2350
   Glu Asp Met Glu Glu Asn
   2355
<210> 15
   <211> 2552
   <212> PRT
   <213> Homo sapiens
<400> 15
   Met Ala Val Glu Thr Arg Ala Glu Leu Val Gly Lys Arg Phe Leu Cys
   1 5 10 15
   Val Ala Val Gly Asp Glu Ala Arg Ser Glu Arg Trp Glu Ser Gly Arg
   20 25 30
   Gly Trp Arg Ser Trp Arg Ala Gly Val Ile Arg Ala Val Ser His Arg
   35 40 45
   Asp Ser Arg Asn Pro Asp Leu Ala Met Ile Gln Ile Ser Gly Asn Gly
   50 55 60
   Asn Ser Ser Gly Val Tyr Val Glu Phe Asp Asp Leu Glu Trp Asp Lys
   65 70 75 80
   Arg Glu Trp Val Lys Val Tyr Glu Asp Phe Ser Thr Phe Leu Val Glu
   85 90 95
   Tyr His Leu Ile Trp Ala Lys Arg Asn Asp Pro Ser Gln Thr Gln Gly
   100 105 110
   Ser Lys Ser Lys Gln Ile Gln Trp Pro Ala Leu Thr Phe Lys Pro Leu
   115 120 125
   Val Glu Arg Asn Ile Pro Ser Ser Val Thr Ala Val Glu Phe Leu Val
   130 135 140
   Asp Lys Gln Leu Asp Phe Leu Thr Glu Asp Ser Ala Phe Gln Pro Tyr
   145 150 155 160
   Gln Asp Asp Ile Asp Ser Leu Asn Pro Val Leu Arg Asp Asn Pro Gln
   165 170 175
   Leu His Glu Glu Val Lys Val Trp Val Lys Glu Gln Lys Val Gln Glu
   180 185 190
   Ile Phe Met Gln Gly Pro Tyr Ser Leu Asn Gly Tyr Arg Val Arg Val
   195 200 205
   Tyr Arg Gln Asp Ser Ala Thr Gln Trp Phe Thr Gly Ile Ile Thr His
   210 215 220
   His Asp Leu Phe Thr Arg Thr Met Ile Val Met Asn Asp Gln Val Leu
   225 230 235 240
   Glu Pro Gln Asn Val Asp Pro Ser Met Val Gln Met Thr Phe Leu Asp
   245 250 255
   Asp Val Val His Ser Leu Leu Lys Gly Glu Asn Ile Gly Ile Thr Ser
   260 265 270
   Arg Arg Arg Ser Arg Ala Asn Gln Asn Val Asn Ala Val His Ser His
   275 280 285
   Tyr Thr Arg Ala Gln Ala Asn Ser Pro Arg Pro Ala Met Asn Ser Gln
   290 295 300
   Ala Ala Val Pro Lys Gln Asn Thr His Gln Gln Gln Gln Gln Arg Ser
   305 310 315 320
   Ile Arg Pro Asn Lys Arg Lys Gly Ser Asp Ser Ser Ile Pro Asp Glu
   325 330 335
   Glu Lys Met Lys Glu Glu Lys Tyr Asp Tyr Ile Ser Arg Gly Glu Asn
   340 345 350
   Pro Lys Gly Lys Asn Lys His Leu Met Asn Lys Arg Arg Lys Pro Glu
   355 360 365
   Glu Asp Glu Lys Lys Leu Asn Met Lys Arg Leu Arg Thr Asp Asn Val
   370 375 380
   Ser Asp Phe Ser Glu Ser Ser Asp Ser Glu Asn Ser Asn Lys Arg Ile
   385 390 395 400
   Ile Asp Asn Ser Ser Glu Gln Lys Pro Glu Asn Glu Leu Lys Asn Lys
   405 410 415
   Asn Thr Ser Lys Ile Asn Gly Glu Glu Gly Lys Pro His Asn Asn Glu
   420 425 430
   Lys Ala Gly Glu Glu Thr Leu Lys Asn Ser Gln Pro Pro Trp Asp Gln
   435 440 445
   Ile Gln Glu Asp Lys Lys His Glu Glu Ala Glu Lys Arg Lys Ser Val
   450 455 460
   Asp Thr Gln Leu Gln Glu Asp Met Ile Ile His Ser Ser Glu Gln Ser
   465 470 475 480
   Thr Val Ser Asp His Asn Ser Asn Asp Leu Leu Pro Gln Glu Cys Asn
   485 490 495
   Met Asp Lys Thr His Thr Met Glu Leu Leu Pro Lys Glu Lys Phe Val
   500 505 510
   Ser Arg Pro Pro Thr Pro Lys Cys Val Ile Asp Ile Thr Asn Asp Thr
   515 520 525
   Asn Leu Glu Lys Val Ala Gln Glu Asn Ser Ser Thr Phe Gly Leu Gln
   530 535 540
   Thr Leu Gln Lys Met Asp Pro Asn Val Ser Asp Ser Lys His Ser Ile
   545 550 555 560
   Ala Asn Ala Lys Phe Leu Glu Thr Ala Lys Lys Asp Ser Asp Gln Ser
   565 570 575
   Trp Val Ser Asp Val Val Lys Val Asp Leu Thr Gln Ser Ser Val Thr
   580 585 590
   Asn Ala Ser Ser Gly Asn Asp His Leu Asn Met Glu Lys Glu Lys Tyr
   595 600 605
   Val Ser Tyr Ile Ser Pro Leu Ser Ala Val Ser Val Met Glu Asp Lys
   610 615 620
   Leu His Lys Arg Ser Pro Pro Pro Glu Thr Ile Lys Ser Lys Leu Asn
   625 630 635 640
   Thr Ser Val Asp Thr His Lys Ile Lys Ser Ser Pro Ser Pro Glu Val
   645 650 655
   Val Lys Pro Lys Ile Thr His Ser Pro Asp Ser Val Lys Ser Lys Ala
   660 665 670
   Thr Tyr Val Asn Ser Gln Ala Thr Gly Glu Arg Arg Leu Ala Asn Lys
   675 680 685
   Ile Glu His Glu Leu Ser Arg Cys Ser Phe His Pro Ile Pro Thr Arg
   690 695 700
   Ser Ser Thr Leu Glu Thr Thr Lys Ser Pro Leu Ile Ile Asp Lys Asn
   705 710 715 720
   Glu His Phe Thr Val Tyr Arg Asp Pro Ala Leu Ile Gly Ser Glu Thr
   725 730 735
   Gly Ala Asn His Ile Ser Pro Phe Leu Ser Gln His Pro Phe Pro Leu
   740 745 750
   His Ser Ser Ser His Arg Thr Cys Leu Asn Pro Gly Thr His His Pro
   755 760 765
   Ala Leu Thr Pro Ala Pro His Leu Leu Ala Gly Ser Ser Ser Gln Thr
   770 775 780
   Pro Leu Pro Thr Ile Asn Thr His Pro Leu Thr Ser Gly Pro His His
   785 790 795 800
   Ala Val His His Pro His Leu Leu Pro Thr Val Leu Pro Gly Val Pro
   805 810 815
   Thr Ala Ser Leu Leu Gly Gly His Pro Arg Leu Glu Ser Ala His Ala
   820 825 830
   Ser Ser Leu Ser His Leu Ala Leu Ala His Gln Gln Gln Gln Gln Leu
   835 840 845
   Leu Gln His Gln Ser Pro His Leu Leu Gly Gln Ala His Pro Ser Ala
   850 855 860
   Ser Tyr Asn Gln Leu Gly Leu Tyr Pro Ile Ile Trp Gln Tyr Pro Asn
   865 870 875 880
   Gly Thr His Ala Tyr Ser Gly Leu Gly Leu Pro Ser Ser Lys Trp Val
   885 890 895
   His Pro Glu Asn Ala Val Asn Ala Glu Ala Ser Leu Arg Arg Asn Ser
   900 905 910
   Pro Ser Pro Trp Leu His Gln Pro Thr Pro Val Thr Ser Ala Asp Gly
   915 920 925
   Ile Gly Leu Leu Ser His Ile Pro Val Arg Pro Ser Ser Ala Glu Pro
   930 935 940
   His Arg Pro Leu Lys Ile Thr Ala His Ser Ser Pro Pro Leu Thr Lys
   945 950 955 960
   Thr Leu Val Asp His His Lys Glu Glu Leu Glu Arg Lys Ala Phe Met
   965 970 975
   Glu Pro Leu Arg Ser Val Ala Ser Thr Ser Ala Lys Asn Asp Leu Asp
   980 985 990
   Leu Asn Arg Ser Gln Thr Gly Lys Asp Cys His Leu His Arg His Phe
   995 1000 1005
   Val Asp Pro Val Leu Asn Gln Leu Gln Arg Pro Pro Gln Glu Thr Gly
   1010 1015 1020
   Glu Arg Leu Asn Lys Tyr Lys Glu Glu His Arg Arg Ile Leu Gln Glu
   1025 1030 1035 1040
   Ser Ile Asp Val Ala Pro Phe Thr Thr Lys Ile Lys Gly Leu Glu Gly
   1045 1050 1055
   Glu Arg Glu Asn Tyr Ser Arg Val Ala Ser Ser Ser Ser Ser Pro Lys
   1060 1065 1070
   Ser His Ile Ile Lys Gln Asp Met Asp Val Glu Arg Ser Val Ser Asp
   1075 1080 1085
   Leu Tyr Lys Met Lys His Ser Val Pro Gln Ser Leu Pro Gln Ser Asn
   1090 1095 1100
   Tyr Phe Thr Thr Leu Ser Asn Ser Val Val Asn Glu Pro Pro Arg Ser
   1105 1110 1115 1120
   Tyr Pro Ser Lys Glu Val Ser Asn Ile Tyr Gly Asp Lys Gln Ser Asn
   1125 1130 1135
   Ala Leu Ala Ala Ala Ala Ala Asn Pro Gln Thr Leu Thr Ser Phe Ile
   1140 1145 1150
   Thr Ser Leu Ser Lys Pro Pro Pro Leu Ile Lys His Gln Pro Glu Ser
   1155 1160 1165
   Glu Gly Leu Val Gly Lys Ile Pro Glu His Leu Pro His Gln Ile Ala
   1170 1175 1180
   Ser His Ser Val Thr Thr Phe Arg Asn Asp Cys Arg Ser Pro Thr His
   1185 1190 1195 1200
   Leu Thr Val Ser Ser Thr Asn Thr Leu Arg Ser Met Pro Ala Leu His
   1205 1210 1215
   Arg Ala Pro Val Phe His Pro Pro Ile His His Ser Leu Glu Arg Lys
   1220 1225 1230
   Glu Gly Ser Tyr Ser Ser Leu Ser Pro Pro Thr Leu Thr Pro Val Met
   1235 1240 1245
   Pro Val Asn Ala Gly Gly Lys Val Gln Glu Ser Gln Lys Pro Pro Thr
   1250 1255 1260
   Leu Ile Pro Glu Pro Lys Asp Ser Gln Ala Asn Phe Lys Ser Ser Ser
   1265 1270 1275 1280
   Glu Gln Ser Leu Thr Glu Met Trp Arg Pro Asn Asn Asn Leu Ser Lys
   1285 1290 1295
   Glu Lys Thr Glu Trp His Val Glu Lys Ser Ser Gly Lys Leu Gln Ala
   1300 1305 1310
   Ala Met Ala Ser Val Ile Val Arg Pro Ser Ser Ser Thr Lys Thr Asp
   1315 1320 1325
   Ser Met Pro Ala Met Gln Leu Ala Ser Lys Asp Arg Val Ser Glu Arg
   1330 1335 1340
   Ser Ser Ala Gly Ala His Lys Thr Asp Cys Leu Lys Leu Ala Glu Ala
   1345 1350 1355 1360
   Gly Glu Thr Gly Arg Ile Ile Leu Pro Asn Val Asn Ser Asp Ser Val
   1365 1370 1375
   His Thr Lys Ser Glu Lys Asn Phe Gln Ala Val Ser Gln Gly Ser Val
   1380 1385 1390
   Pro Ser Ser Val Met Ser Ala Val Asn Thr Met Cys Asn Thr Lys Thr
   1395 1400 1405
   Asp Val Ile Thr Ser Ala Ala Asp Thr Thr Ser Val Ser Ser Trp Gly
   1410 1415 1420
   Gly Ser Glu Val Ile Ser Ser Leu Ser Asn Thr Ile Leu Ala Ser Thr
   1425 1430 1435 1440
   Ser Ser Glu Cys Val Ser Ser Lys Ser Val Ser Gln Pro Val Ala Gln
   1445 1450 1455
   Lys Gln Glu Cys Lys Val Ser Thr Thr Ala Pro Val Thr Leu Ala Ser
   1460 1465 1470
   Ser Lys Thr Gly Ser Val Val Gln Pro Ser Ser Gly Phe Ser Gly Thr
   1475 1480 1485
   Thr Asp Phe Ile His Leu Lys Lys His Lys Ala Ala Leu Ala Ala Ala
   1490 1495 1500
   Gln Tyr Lys Ser Ser Asn Ala Ser Glu Thr Glu Pro Asn Ala Ile Lys
   1505 1510 1515 1520
   Asn Gln Thr Leu Ser Ala Ser Leu Pro Leu Asp Ser Thr Val Ile Cys
   1525 1530 1535
   Ser Thr Ile Asn Lys Ala Asn Ser Val Gly Asn Gly Gln Ala Ser Gln
   1540 1545 1550
   Thr Ser Gln Pro Asn Tyr His Thr Lys Leu Lys Lys Ala Trp Leu Thr
   1555 1560 1565
   Arg His Ser Glu Glu Asp Lys Asn Thr Asn Lys Met Glu Asn Ser Gly
   1570 1575 1580
   Asn Ser Val Ser Glu Ile Ile Lys Pro Cys Ser Val Asn Leu Ile Ala
   1585 1590 1595 1600
   Ser Thr Ser Ser Asp Ile Gln Asn Ser Val Asp Ser Lys Ile Ile Val
   1605 1610 1615
   Asp Lys Tyr Val Lys Asp Asp Lys Val Asn Arg Arg Lys Ala Lys Arg
   1620 1625 1630
   Thr Tyr Glu Ser Gly Ser Glu Ser Gly Asp Ser Asp Glu Ser Glu Ser
   1635 1640 1645
   Lys Ser Glu Gln Arg Thr Lys Arg Gln Pro Lys Pro Thr Tyr Lys Lys
   1650 1655 1660
   Lys Gln Asn Asp Leu Gln Lys Arg Lys Gly Glu Ile Glu Glu Asp Leu
   1665 1670 1675 1680
   Lys Pro Asn Gly Val Leu Ser Arg Ser Ala Lys Glu Arg Ser Lys Leu
   1685 1690 1695
   Lys Leu Gln Ser Asn Ser Asn Thr Gly Ile Pro Arg Ser Val Leu Lys
   1700 1705 1710
   Asp Trp Arg Lys Val Lys Lys Leu Lys Gln Thr Gly Glu Ser Phe Leu
   1715 1720 1725
   Gln Asp Asp Ser Cys Cys Glu Ile Gly Pro Asn Leu Gln Lys Cys Arg
   1730 1735 1740
   Glu Cys Arg Leu Ile Arg Ser Lys Lys Gly Glu Glu Pro Ala His Ser
   1745 1750 1755 1760
   Pro Val Phe Cys Arg Phe Tyr Tyr Phe Arg Arg Leu Ser Phe Ser Lys
   1765 1770 1775
   Asn Gly Val Val Arg Ile Asp Gly Phe Ser Ser Pro Asp Gln Tyr Asp
   1780 1785 1790
   Asp Glu Ala Met Ser Leu Trp Thr His Glu Asn Phe Glu Asp Asp Glu
   1795 1800 1805
   Leu Asp Ile Glu Thr Ser Lys Tyr Ile Leu Asp Ile Ile Gly Asp Lys
   1810 1815 1820
   Phe Cys Gln Leu Val Thr Ser Glu Lys Thr Ala Leu Ser Trp Val Lys
   1825 1830 1835 1840
   Lys Asp Ala Lys Ile Ala Trp Lys Arg Ala Val Arg Gly Val Arg Glu
   1845 1850 1855
   Met Cys Asp Ala Cys Glu Ala Thr Leu Phe Asn Ile His Trp Val Cys
   1860 1865 1870
   Gln Lys Cys Gly Phe Val Val Cys Leu Asp Cys Tyr Lys Ala Lys Glu
   1875 1880 1885
   Arg Lys Ser Ser Arg Asp Lys Glu Leu Tyr Ala Trp Met Lys Cys Val
   1890 1895 1900
   Lys Gly Gln Pro His Asp His Lys His Leu Met Pro Thr Gln Ile Ile
   1905 1910 1915 1920
   Pro Gly Ser Val Leu Thr Asp Leu Leu Asp Ala Met His Thr Leu Arg
   1925 1930 1935
   Glu Lys Tyr Gly Ile Lys Ser His Cys His Cys Thr Asn Lys Gln Asn
   1940 1945 1950
   Leu Gln Val Gly Asn Phe Pro Thr Met Asn Gly Val Ser Gln Val Leu
   1955 1960 1965
   Gln Asn Val Leu Asn His Ser Asn Lys Ile Ser Leu Cys Met Pro Glu
   1970 1975 1980
   Ser Gln Gln Gln Asn Thr Pro Pro Lys Ser Glu Lys Asn Gly Gly Ser
   1985 1990 1995 2000
   Ser Pro Glu Ser Asp Val Gly Thr Asp Asn Lys Leu Thr Pro Pro Glu
   2005 2010 2015
   Ser Gln Ser Pro Leu His Trp Leu Ala Asp Leu Ala Glu Gln Lys Ala
   2020 2025 2030
   Arg Glu Glu Lys Lys Glu Asn Lys Glu Leu Thr Leu Glu Asn Gln Ile
   2035 2040 2045
   Lys Glu Glu Arg Glu Gln Asp Asn Ser Glu Ser Pro Asn Gly Arg Thr
   2050 2055 2060
   Ser Pro Leu Val Ser Gln Asn Asn Glu Gln Gly Ser Thr Leu Arg Asp
   2065 2070 2075 2080
   Leu Leu Thr Thr Thr Ala Gly Lys Leu Arg Val Gly Ser Thr Asp Ala
   2085 2090 2095
   Gly Ile Ala Phe Ala Pro Val Tyr Ser Met Gly Ala Pro Ser Ser Lys
   2100 2105 2110
   Ser Gly Arg Thr Met Pro Asn Ile Leu Asp Asp Ile Ile Ala Ser Val
   2115 2120 2125
   Val Glu Asn Lys Ile Pro Pro Ser Lys Thr Ser Lys Ile Asn Val Lys
   2130 2135 2140
   Pro Glu Leu Lys Glu Glu Pro Glu Glu Ser Ile Ile Ser Ala Val Asp
   2145 2150 2155 2160
   Glu Asn Asn Lys Leu Tyr Ser Asp Ile Pro His Ser Trp Ile Cys Glu
   2165 2170 2175
   Lys His Ile Leu Trp Leu Lys Asp Tyr Lys Asn Ser Ser Asn Trp Lys
   2180 2185 2190
   Leu Phe Lys Glu Cys Trp Lys Gln Gly Gln Pro Ala Val Val Ser Gly
   2195 2200 2205
   Val His Lys Lys Met Asn Ile Ser Leu Trp Lys Ala Glu Ser Ile Ser
   2210 2215 2220
   Leu Asp Phe Gly Asp His Gln Ala Asp Leu Leu Asn Cys Lys Asp Ser
   2225 2230 2235 2240
   Ile Ile Ser Asn Ala Asn Val Lys Glu Phe Trp Asp Gly Phe Glu Glu
   2245 2250 2255
   Val Ser Lys Arg Gln Lys Asn Lys Ser Gly Glu Thr Val Val Leu Lys
   2260 2265 2270
   Leu Lys Asp Trp Pro Ser Gly Glu Asp Phe Lys Thr Met Met Pro Ala
   2275 2280 2285
   Arg Tyr Glu Asp Leu Leu Lys Ser Leu Pro Leu Pro Glu Tyr Cys Asn
   2290 2295 2300
   Pro Glu Gly Lys Phe Asn Leu Ala Ser His Leu Pro Gly Phe Phe Val
   2305 2310 2315 2320
   Arg Pro Asp Leu Gly Pro Arg Leu Cys Ser Ala Tyr Gly Val Val Ala
   2325 2330 2335
   Ala Lys Asp His Asp Ile Gly Thr Thr Asn Leu His Ile Glu Val Ser
   2340 2345 2350
   Asp Val Val Asn Ile Leu Val Tyr Val Gly Ile Ala Lys Gly Asn Gly
   2355 2360 2365
   Ile Leu Ser Lys Ala Gly Ile Leu Lys Lys Phe Glu Glu Glu Asp Leu
   2370 2375 2380
   Asp Asp Ile Leu Arg Lys Arg Leu Lys Asp Ser Ser Glu Ile Pro Gly
   2385 2390 2395 2400
   Ala Leu Trp His Ile Tyr Ala Gly Lys Asp Val Asp Lys Ile Arg Glu
   2405 2410 2415
   Phe Leu Gln Lys Ile Ser Lys Glu Gln Gly Leu Glu Val Leu Pro Glu
   2420 2425 2430
   His Asp Pro Ile Arg Asp Gln Ser Trp Tyr Val Asn Lys Lys Leu Arg
   2435 2440 2445
   Gln Arg Leu Leu Glu Glu Tyr Gly Val Arg Thr Cys Thr Leu Ile Gln
   2450 2455 2460
   Phe Leu Gly Asp Ala Ile Val Leu Pro Ala Gly Ala Leu His Gln Val
   2465 2470 2475 2480
   Gln Asn Phe His Ser Cys Ile Gln Val Thr Glu Asp Phe Val Ser Pro
   2485 2490 2495
   Glu His Leu Val Glu Ser Phe His Leu Thr Gln Glu Leu Arg Leu Leu
   2500 2505 2510
   Lys Glu Glu Ile Asn Tyr Asp Asp Lys Leu Gln Val Lys Asn Ile Leu
   2515 2520 2525
   Tyr His Ala Val Lys Glu Met Val Arg Ala Leu Lys Ile His Glu Asp
   2530 2535 2540
   Glu Val Glu Asp Met Glu Glu Asn
   2545 2550
<210> 18
   <211> 953
   <212> PRT
   <213> Homo sapiens
<400> 18
   Met Pro Ala Met Gln Leu Ala Ser Lys Asp Arg Val Ser Glu Arg Ser
   1               5                   10                  15
   Ser Ala Gly Ala His Lys Thr Asp Cys Leu Lys Leu Ala Glu Ala Gly
               20                  25                  30
   Glu Thr Gly Arg Ile Ile Leu Pro Asn Val Asn Ser Asp Ser Val His
           35                  40                  45
   Thr Lys Ser Glu Lys Asn Phe Gln Ala Val Ser Gln Gly Ser Val Pro
       50                  55                  60
   Ser Ser Val Met Ser Ala Val Asn Thr Met Cys Asn Thr Lys Thr Asp
   65                  70                  75                  80
   Val Ile Thr Ser Ala Ala Asp Thr Thr Ser Val Ser Ser Trp Gly Gly
                   85                  90                  95
   Ser Glu Val Ile Ser Ser Leu Ser Asn Thr Ile Leu Ala Ser Thr Ser
   100 105 110
   Ser Glu Cys Val Ser Ser Lys Ser Val Ser Gln Pro Val Ala Gln Lys
   115 120 125
   Gln Glu Cys Lys Val Ser Thr Thr Ala Pro Val Thr Leu Ala Ser Ser
   130 135 140
   Lys Thr Gly Ser Val Val Gln Pro Ser Ser Gly Phe Ser Gly Thr Thr
   145 150 155 160
   Asp Phe Ile His Leu Lys Lys His Lys Ala Ala Leu Ala Ala Ala Gln
   165 170 175
   Tyr Lys Ser Ser Asn Ala Ser Glu Thr Glu Pro Asn Ala Ile Lys Asn
   180 185 190
   Gln Thr Leu Ser Ala Ser Leu Pro Leu Asp Ser Thr Val Ile Cys Ser
   195 200 205
   Thr Ile Asn Lys Ala Asn Ser Val Gly Asn Gly Gln Ala Ser Gln Thr
   210 215 220
   Ser Gln Pro Asn Tyr His Thr Lys Leu Lys Lys Ala Trp Leu Thr Arg
   225 230 235 240
   His Ser Glu Glu Asp Lys Asn Thr Asn Lys Met Glu Asn Ser Gly Asn
   245 250 255
   Ser Val Ser Glu Ile Ile Lys Pro Cys Ser Val Asn Leu Ile Ala Ser
   260 265 270
   Thr Ser Ser Asp Ile Gln Asn Ser Val Asp Ser Lys Ile Ile Val Asp
   275 280 285
   Lys Tyr Val Lys Asp Asp Lys Val Asn Arg Arg Lys Ala Lys Arg Thr
   290 295 300
   Tyr Glu Ser Gly Ser Glu Ser Gly Asp Ser Asp Glu Ser Glu Ser Lys
   305 310 315 320
   Ser Glu Gln Arg Thr Lys Arg Gln Pro Lys Pro Thr Tyr Lys Lys Lys
   325 330 335
   Gln Asn Asp Leu Gln Lys Arg Lys Gly Glu Ile Glu Glu Asp Leu Lys
   340 345 350
   Pro Asn Gly Val Leu Ser Arg Ser Ala Lys Glu Arg Ser Lys Leu Lys
   355 360 365
   Leu Gln Ser Asn Ser Asn Thr Lys Ile Ala Trp Lys Arg Ala Val Arg
   370 375 380
   Gly Val Arg Glu Met Cys Asp Ala Cys Glu Ala Thr Leu Phe Asn Ile
   385 390 395 400
   His Trp Val Cys Gln Lys Cys Gly Phe Val Val Cys Leu Asp Cys Tyr
   405 410 415
   Lys Ala Lys Glu Arg Lys Ser Ser Arg Asp Lys Glu Leu Tyr Ala Trp
   420 425 430
   Met Lys Cys Val Lys Gly Gln Pro His Asp His Lys His Leu Met Pro
           435                 440                 445
   Thr Gln Ile Ile Pro Gly Ser Val Leu Thr Asp Leu Leu Asp Ala Met
       450                 455                 460
   His Thr Leu Arg Glu Lys Tyr Gly Ile Lys Ser His Cys His Cys Thr
   465                 470                 475                 480
   Asn Lys Gln Asn Leu Gln Val Gly Asn Phe Pro Thr Met Asn Gly Val
                   485                 490                 495
   Ser Gln Val Leu Gln Asn Val Leu Asn His Ser Asn Lys Ile Ser Leu
               500                 505                 510
   Cys Met Pro Glu Ser Gln Gln Gln Asn Thr Pro Pro Lys Ser Glu Lys
           515                 520                 525
   Asn Gly Gly Ser Ser Pro Glu Ser Asp Val Gly Thr Asp Asn Lys Leu
       530                 535                 540
   Thr Pro Pro Glu Ser Gln Ser Pro Leu His Trp Leu Ala Asp Leu Ala
   545                 550                 555                 560
   Glu Gln Lys Ala Arg Glu Glu Lys Lys Glu Asn Lys Glu Leu Thr Leu
                   565                 570                 575
   Glu Asn Gln Ile Lys Glu Glu Arg Glu Gln Asp Asn Ser Glu Ser Pro
               580                 585                 590
   Asn Gly Arg Thr Ser Pro Leu Val Ser Gln Asn Asn Glu Gln Gly Ser
           595                 600                 605
   Thr Leu Arg Asp Leu Leu Thr Thr Thr Ala Gly Lys Leu Arg Val Gly
       610                 615                 620
   Ser Thr Asp Ala Gly Ile Ala Phe Ala Pro Val Tyr Ser Met Gly Ala
   625                 630                 635                 640
   Pro Ser Ser Lys Ser Gly Arg Thr Met Pro Asn Ile Leu Asp Asp Ile
                   645                 650                 655
   Ile Ala Ser Val Val Glu Asn Lys Ile Pro Pro Ser Lys Thr Ser Lys
               660                 665                 670
   Ile Asn Val Lys Pro Glu Leu Lys Glu Glu Pro Glu Glu Ser Ile Ile
           675                 680                 685
   Ser Ala Val Asp Glu Asn Asn Lys Leu Tyr Ser Asp Ile Pro His Ser
       690                 695                 700
   Trp Ile Cys Glu Lys His Ile Leu Trp Leu Lys Asp Tyr Lys Asn Ser
   705                 710                 715                 720
   Ser Asn Trp Lys Leu Phe Lys Glu Cys Trp Lys Gln Gly Gln Pro Ala
                   725                 730                 735
   Val Val Ser Gly Val His Lys Lys Met Asn Ile Ser Leu Trp Lys Ala
               740                 745                 750
   Glu Ser Ile Ser Leu Asp Phe Gly Asp His Gln Ala Asp Leu Leu Asn
           755                 760                 765
   Cys Lys Asp Ser Ile Ile Ser Asn Ala Asn Val Lys Glu Phe Trp Asp
       770                 775                 780
   Gly Phe Glu Glu Val Ser Lys Arg Gln Lys Asn Lys Ser Gly Glu Thr
   785                 790                 795                 800
   Val Val Leu Lys Leu Lys Asp Trp Pro Ser Gly Glu Asp Phe Lys Thr
                   805                 810                 815
   Met Met Pro Ala Arg Tyr Glu Asp Leu Leu Lys Ser Leu Pro Leu Pro
               820                 825                 830
   Glu Tyr Cys Asn Pro Glu Gly Lys Phe Asn Leu Ala Ser His Leu Pro
           835                 840                 845
   Gly Phe Phe Val Arg Pro Asp Leu Gly Pro Arg Leu Cys Ser Ala Tyr
       850                 855                 860
   Gly Val Val Ala Ala Lys Asp His Asp Ile Gly Thr Thr Asn Leu His
   865                 870                 875                 880
   Ile Glu Val Ser Asp Val Val Asn Ile Leu Val Tyr Val Gly Ile Ala
                   885                 890                 895
   Lys Gly Asn Gly Ile Leu Ser Lys Ala Gly Ile Leu Lys Lys Phe Glu
               900                 905                 910
   Glu Glu Asp Leu Asp Asp Ile Leu Arg Lys Arg Leu Lys Asp Ser Ser
           915                 920                 925
   Glu Ile Pro Gly Ala Leu Trp His Ile Tyr Ala Gly Lys Asp Val Asp
       930                 935                 940
   Lys Ile Arg Glu Phe Leu Gln Lys Val
   945                 950